(19) Europäisches Patentamt
European Patent Office
Office européen des brevets

(11) EP 1 812 120 B1

(12) **EUROPEAN PATENT SPECIFICATION**

(45) Date of publication and mention of the grant of the patent:
**22.04.2015 Bulletin 2015/17**

(21) Application number: **05731222.5**

(22) Date of filing: **26.04.2005**

(51) Int Cl.:
*A61Q 19/00* (2006.01) *A61K 8/73* (2006.01)
*A61L 28/00* (2006.01) *A61Q 17/00* (2006.01)
*A61L 15/28* (2006.01) *C11D 3/22* (2006.01)

(86) International application number:
**PCT/DK2005/000285**

(87) International publication number:
**WO 2005/102262 (03.11.2005 Gazette 2005/44)**

# (54) SKIN-PROTECTING ALKALINITY-CONTROLLING COMPOSITION AND THE USE THEREOF

HAUTSCHÜTZENDE ALKALINITÄT KONTROLLIERENDE ZUSAMMENSETZUNG UND IHRE VERWENDUNG

COMPOSITION DE CONTROLE DE L'ALCALINITE A PROTECTION POUR LA PEAU ET SON UTILISATION

(84) Designated Contracting States:
**AT BE BG CH CY CZ DE DK EE ES FI FR GB GR HU IE IS IT LI LT LU MC NL PL PT RO SE SI SK TR**

(30) Priority: **26.04.2004 DK 200400649**

(43) Date of publication of application:
**01.08.2007 Bulletin 2007/31**

(60) Divisional application:
**10154017.7 / 2 196 242**

(73) Proprietor: **CP Kelco ApS**
**4623 Lille Skensved (DK)**

(72) Inventor: **TRUDSØ, Jens, Eskil**
**DK-4000 Roskilde (DK)**

(74) Representative: **Kiernan, Lisa et al**
**Murgitroyd & Company**
**Scotland House**
**165-169 Scotland Street**
**Glasgow G5 8PL (GB)**

(56) References cited:
**EP-A- 0 664 300** **WO-A-02/28447**
**WO-A-89/12648** **WO-A-02/055034**
**WO-A-2004/026317** **WO-A-2005/049100**
**US-A- 4 278 657** **US-A- 4 813 942**
**US-A- 5 384 400** **US-A- 6 051 250**
**US-A- 6 083 540** **US-B1- 6 428 837**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

Technical Field

The present invention relates to a skin-protecting alkalinity-controlling composition as well as the use of a composition comprising at least one carboxylic acid polysaccharide for skin protection and/or alkalinity control.

Background Art

Pectin is a complex polysaccharide associated with plant cell walls. It consists of an alpha 1-4 linked polygalacturonic acid backbone intervened by rhamnose residues and modified with neutral sugar side chains and non-sugar components such as acetyl, methyl, and ferulic acid groups.

The neutral sugar side chains, which include arabinan and arabinogalactans, are attached to the rhamnose residues in the backbone. The rhamnose residues tend to cluster together on the backbone. So, with the side chains attached, this region is referred to as the hairy region and the rest of the backbone is hence named the smooth region.

In US 5,929,051, Ni, et al. pectin is described as a plant cell wall component. The cell wall is divided into three layers, middle lamella, primary, and secondary cell wall. The middle lamella is the richest in pectin. Pectins are produced and deposited during cell wall growth. Pectins are particularly abundant in soft plant tissues under conditions of fast growth and high moisture content. In cell walls, pectins are present in the form of a calcium complex. The involvement of calcium cross-linking is substantiated by the fact that chelating agents facilitate the release of pectin from cell walls as disclosed by Nanji (US 1,634,879) and Maclay (US 2,375,376). According to Dumitriu, S.: Polysaccharides, Structural diversity and functional versatility, Marcel Dekker, Inc., New York, 1998, 416 - 419, pectin is used in a range of food products.

Historically, pectin has mainly been used as a gelling agent for jam or similar, fruit-containing, or fruit-flavoured, sugar-rich systems. Examples are traditional jams, jams with reduced sugar content, clear jellies, fruit-flavoured confectionery gels, non-fruit-flavoured confectionery gels, heat-reversible glazing for the bakery industry, heat-resistant jams for the bakery industry, ripples for use in ice cream, and fruit preparations for yoghurt.

A substantial portion of pectin is used today for stabilization of low-pH milk drinks, including fermented drinks and mixtures of fruit juice and milk.

The galacturonic acid residues in pectin are partly esterified and present as the methyl ester. The degree of esterification is defined as the percentage of carboxyl groups esterified. Pectin with a degree of esterification ("DE") above 50% is named high methyl ester ("HM") pectin or high ester pectin and one with a DE lower than 50% is referred to as low methyl ester ("LM") pectin or low ester pectin. Most pectins found in plant material such as fruits, vegetables and eelgrass are HM pectins.

Pectins are soluble in water and insoluble in most organic solvents. Pectins with a very low level of methyl-esterification and pectic acids are for practical purposes only soluble as the potassium or sodium salts.

Pectins are most stable at pH 3-4. Below pH 3, methoxyl and acetyl groups and neutral sugar side chains are removed. At elevated temperatures, these reactions are accelerated and cleavage of glycosidic bonds in the galacturonan backbone occurs. Under neutral and alkaline conditions, methyl ester groups are saponified and the polygalacturonan backbone breaks through beta-elimination-cleavage of glycosidic bonds at the non-reducing ends of methoxylated galacturonic acid residues. These reactions also proceed faster with increasing temperature. Pectic acids and LM pectins are resistant to neutral and alkaline conditions since there are no or only limited numbers of methyl ester groups.

Pectin is a weak acid, and is less soluble at low pH than at high pH. Thus, by changing the pH of the pectin during manufacture thereof, a pectin having lower or higher solubility is provided. The pH is typically increased through the use of bases such as alkali metal hydroxides or alkali metal carbonates, but other bases are equally useable. For instance, by using sodium carbonate, sodium pectinate is formed and the higher the dosage of sodium carbonate and, thus, the higher the pH, the more of the carboxylic acids are transformed to their sodium salts.

However, at higher pH the pectin starts to de-esterify during pH-adjustment, handling and storage. Thus the pH should be maintained at a level at or below pH 6.

In some cases, pectin as manufactured is esterified in a block-wise fashion. WO 2004020472 describes this phenomenon as the block-wise de-esterification takes place in the raw material used for making pectin, and the disclosure relates to a method for eliminating this block-wise de-esterification.

WO 8912648 discloses a method for transforming block-wise de-esterified pectin into pectin with a random distribution of ester groups. The method involves the use of polygalacturonase, which splits the pectin molecule in those areas of the pectin molecule that are non-esterified. Thus, this method provides a lower molecular weight pectin having a higher degree of esterification than the block-wise esterified starting pectin.

According to Kertesz, Z. I: The Pectic Substances, Interscience Publishers, Inc, New York, 1951, pectic materials occur in all plant tissues. However, apples, beets, flax, grapefruit, lemons, limes, oranges, potatoes, and sunflower are of particular industrial importance. Lately, also the pectin in Aloe Vera has shown industrial utility.

Pectin according to the present invention needs not be extracted from the pectin containing starting material. Such crude pectin preparations are disclosed in US 2,132,065, US 3,982,003, US 4,831,127, WO 9115517, US 5,354,851, US 5,403,612, US 5,567,462, US 5,656,734, and WO 9749734.

Other esterified carboxy acid polymers include, but are not limited to

- Pectin ethyl ester, made using ethyl iodide and heating as disclosed by Kertesz, Z.I.: The Pectic Substances, Interscience Publishers, Inc., New York, 251, 1951. In addition, pectic acid and pectinic acid may be totally or partially esterified with aliphatic, arylaliphatic, cycloaliphatic or heterocyclic alcohols. When the acid is only partially esterified, the remaining free carboxyl groups may be salified with inorganic or organic bases. The esters may be used in the pharmaceutical, biomedical, alimentary and cosmetic fields. The esters may be prepared from a quaternary ammonium salt of pectic acid or pectinic acid and an esterifying agent such as a halogenide as disclosed in US 5,384,400.

- Esterified polysaccharide manufactured with a ketene dimer using an enzyme as a catalyst under mild reaction conditions as disclosed in US 6,624,298. The polysaccharide used is at least one selected from the group consisting of cellulose ethers, hydroxyethylcellulose, hydroxypropylcellulose, carboxymethylcellulose, guar, cationic guar, and hydroxypropylguar.

- Starch esters. Methods for the preparation of starch esters are described in the article by Tessler, M. M. and Bilimers, R. L., Preparation of Starch Esters, in Journal of Environmental Polymer Degradation 4 (1996) 85-89 and further disclosed in US 6,605,715.

- Polymerized sugar esters as described in US 5,859,217.

- Esters of alginic acid. Examples include ethylene glycol and propylene glycol esters, methyl ester, homologues of methyl ester, and esters of aromatic, araliphatic, alicyclic and heterocyclic alcohols. Also included are esters deriving from substituted alcohols such as esters of bivalent aliphatic alcohols as disclosed in US 5,416,205.

According to www.smartskincare.com, sweat is a salty, watery solution produced by sweat glands, numerous microscopic channels opening onto the skin surface. As sebum and sweat mix up on the skin surface, they form a protective layer often referred to as the acid mantle. The skin is mildly acidic. In addition to helping protect skin from "the elements" (such as wind or pollutants), the acid mantle also inhibits the growth of harmful bacteria and fungi. If the acid mantle is disrupted or loses its acidity, the skin becomes more prone to damage and infection. The loss of acid mantle is one of the side effects of washing the skin with soaps or detergents of moderate or high strength.

According to US 5,837,254, fungal infections of the vagina or urinary tract are difficult to eradicate and frequently recur but are rarely life threatening. The normal pH of the genital tract is 4.5 to 5, which is maintained by lactobacillus. The absence of lactobacillus and a normal pH promotes candidiasis as well as the herpes virus, birth control pills, a weak immune system, genetic factors, stress and a host of other factors, which foster the growth of yeast and fungal infections of the genital tract. Candida albicans grows readily in a moist environment at a pH of more than 5.

In US 5,972,321 it is stated that aalthough body odor may be partially due to certain chemicals secreted by sebaceous glands and eccrine sweat glands, major axillary (underarm) foul odor is due to secretions of the apocrine glands, which contain special nutrient materials for microorganisms. The apocrine glands secrete a milky fluid that has a pH range of 5 to 6.5 and initially consists of lipids, proteins and carbohydrates. Although gram-positive bacteria, which thrive on substances found on the moist skin surface, appear to be responsible for the production of malodor, the precise mechanisms of odor production are still unclear.

According to US 4,666,707, bath salt compositions are prepared by incorporating perfume, colorant, plant extract, organic acid and so on into an inorganic salt mixture comprising sodium sulfate, borax, sulfur, sodium chloride, carbonate salt, etc., and are used for the purpose of providing the bath with perfume and/or color, or adequately stimulating the skin to thereby promote the blood circulation, the recovery from fatigue and/or the metabolism. Among such bath salt compositions, there are foaming bath salt compositions comprising a combination of a carbonate salt and an acid, which produces, in the bath, carbon dioxide gas bubbles to thereby cause a relaxing or refreshing sensation and render bathing enjoyable.

According to US 6,589,923 and US 4,335,025, upon washing with soap, a pH of 8-10 is established in the wash liquor. This alkalinity neutralizes the natural acid mantle of the skin (pH 5-6). Although in normal skin this acid mantle is reformed relatively quickly, in sensitive or pre-damaged skin irritations may result. A further disadvantage of soaps is the formation of insoluble lime soaps in hard water. Being alkaline, soap emulsifies the oily layer covering the natural horny layer (stratum corneum) of a person’s skin and neutralizes a likewise natural acid mantle of the epidermis, which has, normally, an acid pH of approximately 5.5-6.5. Failure to readily regenerate the acid and oily part of the epidermis--particularly among older people-often results in dermatological symptoms, such as itching, chapping and cracking of the epidermis, especially in cold weather. Of course, always to be considered is that significant segment of the population, which is allergic to or cannot tolerate conventional soaps in view of a number of reactions (sensitivities) resulting from the use thereof.

According to US 6,551,987, US 6,013,618 and US 5,626,852, pro-fragrances are compounds, which under certain conditions break down to fragrances. For instance, tris(9-decenyl) when exposed to suitable conditions (e.g., exposure to the acid mantle of human skin) breaks down to release a mixture of 9-decenol and 9-decenyl formate, both of which are fragrance raw materials.

In US 6,352,700 it is stated that while products exist that are said to address the problems of skin irritation and inflamation, they inevitably fail to address the short-term impact of various additives on the pH balance of the skin, i.e., the skin's acid mantle. To put this into perspective, one need only to consider conventional facial tissue, toilet tissue, napkin and paper towel products that are used for wiping dry or wet skin. Upon contact with skin, the tissue products transfer some of the chemicals present in the tissue to the skin surface.

According to US 6,150,405 and US 5,667,769, some hair care preparations particularly for treating hair loss, contain hydroxyl scavengers.

According to US 4,761,279, the application of a conventional shaving preparation of high alkalinity is often irritating to the skin.

US 2,253,389 discloses the use of alkali to make pectin, which does not require sugar and acid to form gels. A gel is formed by soluble pectin in a neutral or slightly alkaline aqueous medium in the presence of a metal compound, and it is stressed that the alkalinity must be insufficient to convert pectin to pectate. The resulting gelling agent is particularly useful for substituting gelatine in jellies of water and milk.

GB 541,528 discloses the importance of applying low temperature for demethoxylating pectin. By controlling alkali hydrolysis of pectin at temperatures between 10°C and the freezing point of the pectin solution, low ester pectin of high setting power and with a low setting temperature can be made. Hydrolysis is performed in an aqueous medium and the hydrolysis is terminated by neutralization. It is disclosed that the hydrolysis is very rapid at pH 12 and very slow at pH 8.5.

US 2,478,170 discloses pectin with 20-30% remaining acid groups, which gel by the addition of calcium ions, with or without sugar. Alkalis are alkali metal hydroxides, ammonium hydroxide, sodium carbonate, organic ammonium bases etc. and the process involves an aqueous solution or extract of pectin being adjusted to temperatures below 35°C and pH 10-12. When the desired methoxyl content is reached, pH is reduced to 4, and the pectinic acid is isolated.

In "The Pectic Substances", Interscience Publishers, Inc., New York, 1951, Kertesz describes the effect of bases on pectin. When alkali is added to a pectin solution to an extent, which is higher than the amount needed for neutralizing the pectin, demethoxylation commences. This process consumes the alkali and the pH of the solution soon drops. Kertesz also refers to other findings, which suggest that the consumption of alkali increases as a result of the alkali concentration, or the duration of treatment with alkali, or as the temperature of the reaction is raised. Thus, he suggests that this alkali consumption may be utilized for determining the ester content of pectinic acids.

JP 2001226220 discloses the use of alcohol extracted Citrus junos seed pectin to make a skin lotion composed of said pectin, deep sea layer water and sea water or water. The lotion is characterized by being non-sticky, non-irritant and by having a low pH. Conventionally, pectin is extracted in water, whereas alcohol is known to make pectin insoluble. In addition, the disclosure does not discuss the composition of the pectin.

WO 02/14374 discloses the use of hydrocolloids as thickening or emulsifying agents for a variety of products, such as foodstuffs, pharmaceutical compositions, personal care products and beverages.

WO 04/005352 discloses the use of amidated pectins, such as in crèmes, lotions and household products.

US 6,509,311 discloses a gel system comprising propylene glycol alginate as a gelling agent, as a water binder, as an emulsifier and as a stabiliser.

The prior art discloses several carboxylic acid polysaccharide compositions including cosmetic compositions. Relevant documents are US 5,384,400; US 6,428,837; WO 89/12648; US 4,278,657; WO 02/055034; EP 664,300; WO 2004/026317; US 5,942,478; WO 02/28447 and US 4,813,942. None of these disclose how to solve problems of controlling the alkalinity of skin by sufficient buffering capacity to avoid a major increase of pH.

A need for a composition remains, which is capable of providing buffering, thus avoiding a major increase in the pH of an aqueous system and/or useable for reducing the pH of aqueous systems, in which alkalinity is formed as a result of chemical and/or biological reactions, or as a result of alkalinity being imposed on the aqueous system by the environment. In particular, there is a need for a composition, which will protect the acid mantle, and there is a need for incorporating such a composition in articles, which are in contact with the skin, either human skin or animal skin.

Disclosure of Invention

The present invention thus relates to a skin-protecting alkalinity-controlling composition comprising a mixture of at least one high DE pectin ester having a degree of esterification (DE) in the range from about 70% to about 100%, more preferably from about 80% to about 100%, and at least one low DE carboxylic acid polysaccharide having a degree of esterification (DE) in the range from about 5% to about 40%, more preferably from about 10% to about 35%.

The present invention further relates to the use of a composition comprising a mixture of at least one high DE pectin ester having a degree of esterification (DE) in the range from about 70% to about 100%, more preferably from about 80% to about 100%, and at least one low DE carboxylic acid polysaccharide having a degree of esterification (DE) in the range from about 5% to about 40%, more preferably from about 10% to about 35%, for the preparation of a medicament for protecting the skin acid mantle by controlling alkalinity.

Preferred embodiments of the inventions are set forth in the dependent claims.

The invention is disclosed in more detail in the following by means of the accompanying drawings and exemplary embodiments of preferred embodiments of the invention.

Brief Description of the Drawings

Fig 1.1 shows the alkali consumption of pectins of different molecular weight,

Fig 1.2 shows the pH-drop over time for the above pectins by dissolution at 70° C,

Fig. 1.3 shows the pH-drop over time for the above pectins by dissolution at 20° C,

Fig. 2.1 shows the alkali consumption of pectins of different degrees of esterification (DE),

Fig. 2.2 shows the pH-drop of the above pectins by dissolution at 70° C,

Fig. 2.3 shows the pH-drop of the above pectins by dissolution at 20° C,

Fig. 2.4 shows the initial about 130 minutes pH-drop of the above pectins dissolved either at 70° or at 20° C,

Fig. 3.1 shows the alkali consumption of either a block-wise or randomly esterified pectin of similar DE,

Fig. 3.2 shows the pH-drop of the above pectins dissolved at either 70° or 20° C,

Fig. 3.3 shows the initial about 100 minutes pH-drop of the above pectins,

Fig. 4.1 shows the pH-drop of a pectin held at various temperatures,

Fig. 5.1 shows the effect of multiple alkali dosages to a pectin,

Fig. 6.1 shows the effect of pectin concentration on pH-drop,

Fig. 7.1 shows the pH-drop of ion-exchanged water without addition of pectin or other additions,

Fig. 8.1 shows the alkali consumption of propylene glycol alginates (PGA) of different degrees of esterification,

Fig. 8.2 shows the pH-drop of the above PGAs by dissolution at 70° C,

Fig. 8.3 shows the pH-drop of the above PGAs by dissolution at 20° C,

Fig. 8.4 shows the initial about 70 minutes pH-drop of the above PGAs by dissolution at either 70° or 20° C,

Fig. 9.1 shows the effect of multiple alkali dosages to propylene glycol alginate,

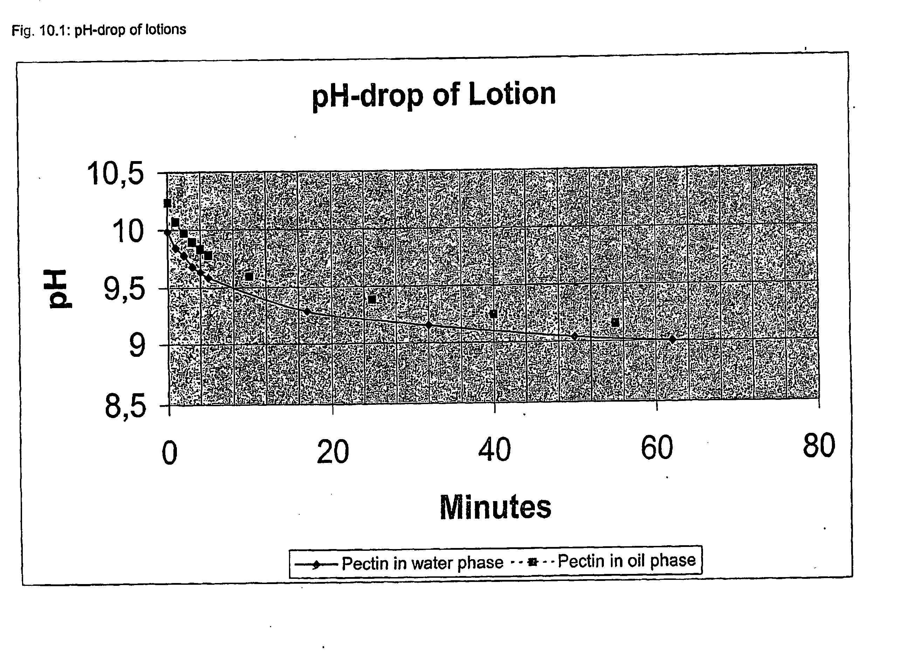

Fig. 10.1 shows the pH-drop of lotions containing pectin in either the water phase or the oil phase,

Fig. 11.1 shows the pH-drop of cloth soaked in a solution of 0.01 % pectin of different molecular weights,

Fig. 11.2 shows the pH-drop of cloth soaked in a solution of 0.05 % pectin of different molecular weights,

Fig. 11.3 shows the pH-drop of cloth soaked in a solution of 0.10 % pectin of different molecular weights,

Fig. 11.4 shows the pH-drop of cloth soaked in a solution of 0.20 % pectin of different molecular weights, and

Fig. 11.5 shows the pH-drop of cloth soaked in a solution of 0.50 % pectin of different molecular weights.

Fig. 12.1 shows the alkali consumption of a mixture of 50% of a pectin having a DE of 93.4% and 50% of a pectin having a DE of 9.6% dissolved at 70 °C and compared with the alkali consumption of the individual components.

Fig. 12.2 shows the pH-drop over time of the above mixture dissolved at 70 °C and compared with the pH-drop of the individual components.

Fig. 13.1 shows the alkali consumption of a mixture of 50% of a pectin having a DE of 93.4% and 50% of a propylene glycol alginate (PGA) having a DE of 55% dissolved at 70 °C and compared with the alkali consumption of the individual components.

Fig. 13.2 shows the pH-drop over time of the above mixture dissolved at 70 °C and compared with the pH-drop of the individual components.

Fig. 14.1 shows the alkali consumption of a mixture of 50% of a propylene glycol alginate (PGA) having a DE of 85% and 50% of a pectin having a DE of 9.6% dissolved at 70 °C and compared with the alkali consumption of the individual components.

Fig. 14.2 shows the pH-drop over time of the above mixture dissolved at 70 °C and compared with the pH-drop of the individual components.

<u>Best Modes for Currying out the Invention</u>

A high DE carboxylic acid polysaccharide provides for a rapid pH-drop due to the low amount of free carboxylic acid groups present. Thus, if a rapid pH-drop is needed, a high DE carboxylic acid polysaccharide should be used. This fact can be utilized in a range of products intended to be applied to the skin of humans or animals. Uses include but are not limited to lotions, creams, foundations, face masks, hair care products, genital lotions, deodorants, ostomy products, feminine hygiene products, laundry products, bath salt products, soap products, fragrance products, lotionized tissue products, and shaving products. Further, such pectin can be used in similar products to treat animals.

According to the invention, said high DE carboxylic acid polysaccharide is a pectin ester, preferably a pectin ester of aliphatic, arylaliphatic, cycloaliphatic or heterocyclic alcohols, more preferably an ester of methanol, ethanol, propanol or isopropanol, and most preferably an ester of methanol.

The advantage of using methanol esters of pectin is the natural occurrence of such ester. However, without being bound by theory, methyl esters of pectin are more prone to liberate the alcohol part thereof during de-esterification. Esters of pectin with higher alcohols are not as prone to alkaline de-esterification.

The pectin is of a molecular weight in the range from about 5,000 to about 140,000, preferably in the range from about 10,000 to about 125,000, most preferably in the range from about 10,000 to about 40,000.

As demonstrated in example 1 below, the molecular weight of pectin has no influence on the alkali consumption or on the pH drop encountered. However, by adjusting the molecular weight of the pectin it is possible to adjust the amount of pectin, which may be dissolved or suspended in a final product. Thus, as disclosed in more detail in example 11, a lower molecular weight pectin is easier to dissolve and the viscosity of the resulting pectin-containing solution is lower than in a corresponding higher molecular weight-containing pectin. This fact can be utilized to obtain a relatively highly concentrated pectin-solution having suitably low viscosity, e.g. for use in fabric-treating products. The pectin having a molecular weight below about 40,000 can be made at concentrations above about 10% without causing unacceptable high viscosity. Such pectin could be manufactured and marketed as a concentrated solution with a pectin concentration in excess of 10%. Alternatively, the possibility of making such pectin solution in concentrations above about 10% makes spray-drying of such solutions economically feasible.

The degree of esterification indicates the average DE of any given polysaccharide. By controlling the distribution of ester groups along the polysaccharide chain to obtain either a random or a block-wise distribution of ester groups, it is possible to obtain a locally higher or lower DE polysaccharide. As demonstrated in example 3, the alkali consumption of a pectin having a block-wise ester group distribution is the same as the alkali consumption of a corresponding pectin having a random ester group distribution. However, the pH-drop of the two pectins is considerably larger for the block-wise esterified pectin, presumably because such pectin will act as a pectin with a higher average DE. Thus, by treating a block-wise esterified pectin with a polygalacturarase, which splits the pectin at non-esterified sites, a lower molecular weight pectin may be obtained having an increased DE.

According to the invention, the skin-protecting alkalinity controlling composition comprises a mixture of at least one high DE pectin ester having a degree of esterification (DE) in the range from about 70% to about 100%, more preferably from about 80% to about 100%, and at least one low DE carboxylic acid polysaccharide having a degree of esterification (DE) in the range from about 5 to about 40 %, but may be from about 10% to about 35%.

A carboxylic acid polysaccharide having a relatively low DE provides for a large alkali consumption capacity or buffer capacity.

An advantage of a higher buffer capacity is the ability of the pectin to neutralize an initial high concentration of alkali. This is an advantage particularly when fabrics are insufficiently depleted for alkaline washing powder. Thus, by combining a low DE and a high DE carboxylic acid polysaccharide, an initial alkali consumption buffering can be obtained succeeded by a pH-reduction.

According to the invention said low DE carboxylic acid polysaccharides are selected from the group comprising pectin esters, alginic acid esters, esterified cellulose ethers, esterified hydroxyethylcellulose, esterified carboxymethylcellulose, esterified guar gum, esterified cationic guar gum, esterified hydrocypropyl guar gum, starch esters, and polymerized sugar esters.

The said low DE carboxylic acid polysaccharides may be a pectin ester, preferably a pectin ester of aliphatic, arylaliphatic, cycloaliphatic or heterocyclic alcohols, more preferably an ester of methanol, ethanol, propanol or isopropanol, and most preferably an ester of methanol.

The said low DE carboxylic acid polysaccharides may be a pectin having a molecular weight in the range from about 5,000 to about 140,000, preferably in the range from about 10,000 to about 125,000, most preferably in the range from about 10,000 to about 40,000.

In a further embodiment according to the invention, the ester groups of the high DE pectin esters and said low DE polysaccharides are distributed in a block-wise fashion.

In another embodiment according to the invention, the ester groups of the high DE pectin esters and said low DE polysaccharides are distributed in a random fashion.

In another embodiment according to the invention of the use of a composition, said composition comprises a mixture of at least one high DE pectin ester having a degree of esterification (DE) in the range from about 70% to about 100%, more preferably from about 80% to about 100%, and at least one low DE carboxylic acid polysaccharide having a degree of esterification (DE) in the range from about 5% to about 40%. Most preferably from about 10% to about 35%.

The composition according to the invention is suitable for use in personal care products.

In a preferred embodiment, said products are for use on human skin.

In another embodiment, said products are for use on animal skin.

In a particular embodiment according to the invention, the skin protecting alkalinity-controlling composition is in the form of a product selected from the group consisting of skin creams, skin lotions and soap products. Also described are deodorant products, fragrance products, hair care products, shaving products, and bath salt products.

In another embodiment according to the invention, the skin protecting alkalinity-controlling composition is incorporated into amides in contact with the skin, selected from the group consisting of female hygiene products and diapers.

A particular advantage of the present composition is the fact that they are capable of controlling the alkalinity of the surface, to which they are applied, for a prolonged time. As demonstrated in examples 5 and 8, the carboxylic acid polysaccharides are capable of controlling the alkalinity at multiple challenges of alkalinity. This fact can be utilized in e.g. deodorant products, diapers or female hygiene products, which are repeatedly exposed to sweat that is decomposed by micro-organisms to alkaline substances. Thus, a prolonged effective alkalinity control may be obtained by the products according to the present invention.

In another embodiment according to the invention, the skin-protecting alkalinity-controlling composition is incorporated into articles selected from the group consisting of ostomy products and wound care products.

In ostomy products a low solubility polysaccharide, such as a low solubility pectin, should be used, since the ostomy product should remain insoluble for a longer period of time during flushing by body fluids. In this particular case a combination of a low DE and a low pH pectin would provide for a longer durability of the ostomy product during use.

In a particular embodiment such low solubility low DE pectin should be combined with a higher solubility pectin having a higher DE to maintain a skin pH closer to the optimum skin pH of 5.5.

In still another embodiment according to the invention, the skin-protecting alkalin-ity-controlling composition is incorporated into articles selected from the group consisting of lotionized tissue products, fabric treating products, and laundry rinse products.

Materials and Methods

a) Extraction of pectin

Pectin is extracted using the following steps. The degree of esterification was controlled in the range of about 76% to about 30% through shorter or longer extraction times.

1. 15 litres of water was heated to 70°C in a stainless steel, jacketed vessel having a volume of 18 litres and equipped with a stirrer.
2. 500 g dried citrus peel or dried beet cossets was added to the water, and the pH is adjusted to 1.7 - 1.8 by addition of 62% nitric acid.
3. Extraction was carried out at 70°C for 2 - 24 hours depending on the desired degree of esterification while stirring.
4. After extraction, the content of the vessel was filtered on a Bücher funnel using diatomaceous earth as filter aid.
5. The filtered extract was ion exchanged while stirring by adding 50ml resin (Amberlite SR1L, produced by Rohm&Haas) per litre of filtered extract. While stirring, the ion exchange was carried out during 20 minutes while stirring.
6. The ion exchanged filtrate was filtered on a Bücher funnel equipped with a cloth.
7. The filtered ion exchanged filtrate was precipitated by adding it to three parts of 80% isopropanol while stirring gently.
8. The precipitate was collected on nylon cloth and pressed by hand to remove as much isopropanol as possible.
9. The hand pressed precipitate was washed once in 60% isopropanol and then dried at 70°C in a drying cabinet at atmospheric pressure.
10. After drying, the pectin was milled.

b) Preparation of pectin with degree of esterification below 30%

1. The pressed precipitate made according to the procedure under a) point 8 was suspended in 60% isopropyl alcohol at 5°C.
2. Concentrated NaOH solution was added and the slurry was stirred for about one hour. The amount of NaOH is calculated to produce the desired DE.
3. The pectin solid was separated on nylon cloth, and washed twice in 60% isopropyl alcohol at pH 4.
4. The pectin solid was separated on nylon cloth, dried at 70°C and milled.

c) Preparation of pectin with different molecular weights

1. Pectin extracted according to a) was dissolved in about 80 °C ion exchanged water to form a 5% solution.
2. After cooling the solution to about 25 °C, pH was adjusted to 5.50 with $NH_3$.
3. Samples of the cold solution were treated with pectin lyase in concentrations ranging from 0 to 1300 micro litres per 10 litres of pectin solution.
4. Each sample was treated with its enzyme preparation for 1 hour at 25 °C while stirring.
5. After treatment, the pH was adjusted to 2.50 and the samples were heated at 80 °C for 10 minutes to inactivate the enzyme.
6. The samples were lastly precipitated in isopropyl alcohol, washed in isopropyl alcohol, dried and milled.

d) Preparation of pectin with degrees of esterification above 80%

1. 50 g. pectin as prepared under a) was added 2.5 g. dimethylaminopyridine, 100 ml. methanol and 100 ml. heptane in suitable flask and the mixture was cooled to minus 4 °C.
2. 15 ml thionylchloride was over a period of 10 minutes added as drops to the mixture.
3. Over about 24 hours, the mixture was allowed to heat to about 21°C.
4. The solid was filtered, washed twice with first 60% isopropyl alcohol and secondly with 100% isopropyl alcohol.
5. The solid was dried at about 70°C.

e) Preparation of pectin with different distribution of ester groups

1. Pectin extracted according to a) was dissolved in about 80 °C ion exchanged water to form a 2% solution.
2. The solution was cooled to 45 °C and pH was adjusted to 4.5 with $NH_3$.
3. Samples were added 2 - 4% of enzyme preparation while stirring: Plant esterase (Collopulin) for a block wise de-esterification and bacterial esterase (Rheozyme) for random de-esterification.
4. The degree of esterification was monitored through titration with 2% $NH_3$ at constant pH of 4.5.
5. After de-esterification, decreasing the pH to 2.5 with $HNO_3$ and subsequently heating the sample to 80 °C for 10 minutes inactivated the enzyme.
6. The sample was precipitated in isopropyl alcohol, washed in isopropyl alcohol, dried and milled.

f) Determination of molecular weight (Mw) and intrinsic viscosity (IV)

For this, High Performance Size Exclusion Chromatography (HPSEC) with triple detection is used.

Principle:

A pectin sample is fractionated according to hydrodynamic volume, using size exclusion chromatography. After separation, the sample is analysed by a triple detector system, consisting of a refractive index (RI) detector, a Right Angle Laser Light Scattering (RALLS) detector and a differential viscometer. Information from these detectors leads to determination of molecular weight (Mw) and intrinsic viscosity (IV). The Mark-Houwink factor is calculated using the molecular weight and intrinsic viscosity as obtained using this method.

Materials:

1. Pump model 515, Waters, Hedehusene, Denmark.
2. Degasser, Gynkotek, Polygen Scandinavia, Arhus, Denmark.
3. Column oven, Waters, Hedehusene, Denmark.
4. AS-3500 Auto sampler, with sample preparation module, Dionex Denmark, Rødovre, Denmark.
5. 3 linear mixed bed columns, TSK-GMPWXL, Supelco, Bellefonte PA, USA.
6. Liquid phase: 0.3 M lithium acetate buffer pH 4.8, Fluka Chemie AG, Buchs, Switzerland.
7. Dual detector, RI, Viscometry, Model 250, Viscotek, Houston, Texas, USA.
8. RALLS Model 600, Viscotek, Houston, Texas, USA.

Method:

Approximately 2 mg of the sample is weighed into a 2000 μl vial. The sample is then dissolved in the auto sampler, by following schedule: 8 μl of ethanol is added, then 1300 μl of acetate buffer (0.3 M, pH 4.8), sample is heated to 75 °C and mixed for 9.9 minutes. 300 μl of the preparation is diluted with 900 μl of acetate buffer, then mixing for 9.9 minutes. Sample is left at ambient temperature for 20 minutes. 100 μl of the sample is injected with a 100 μl full loop and flow rate is 0.8 ml/min. Two detectors are present in line, a right angle laser light Scattering (RALLS) detector (Viscotek) and a dual detector consisting of a refractive index detector and a viscometer (Viscotek).

The specific refractive index increment (dn/dc) value for pectin is set at 0.144.

Data from detectors are processed by tri-SEC software (Viscotek).

g) Determination of degree of esterification (DE) and galacturonic acid (GA) in non-amide pectin

Principle:

This method pertains to the determination of % DE and % GA in pectin, which does not contain amide and acetate ester.

Apparatus:

1. Analytical balance
2. Glass beaker, 250 ml, 5 pieces
3. Measuring glass, 100 ml
4. Vacuum pump
5. Suction flask
6. Glass filter crucible no. 1 (Büchner funnel and filter paper)
7. Stop watch
8. Test tube
9. Drying cabinet at 105 °C
10. Dessicator
11. Magnetic stirrer and magnets
12. Burette (10 ml, accuracy ± 0,05 ml)
13. Pipettes (20 ml: 2 pieces, 10 ml: 1 piece)
14. pH-meter/autoburette or phenolphtalein

Chemicals:

1. Carbon dioxide-free water (deionized water)
2. Isopropanol (IPA), 60% and 100%
3. Hydrochloride (HCl), 0.5 N and fuming 37%
4. Sodium hydroxide (NaOH), 0.1 N (corrected to four decimals, e.g. 0.1002), 0.5 N
5. Silver nitrate ($AgNO_3$), 0.1 N
6. Nitric acid ($HNO_3$), 3 N
7. Indicator, phenolphtalein, 0.1%

Procedure - Determination of % DE and % GA
(Acid alcohol: 100 ml 60% IPA + 5 ml HCl fuming 37%):

1. Weigh 2.0000 g pectin in a 250 ml glass beaker.
2. Add 100 ml acid alcohol and stir on a magnetic stirrer for 10 min.
3. Filtrate through a dried, weighed glass filter crucible.
4. Rinse the beaker completely with 6 x 15 ml acid alcohol.
5. Wash with 60% IPA until the filtrate is chloride-free (approximately 500 ml).
6. Wash with 20 ml 100% IPA.
7. Dry the sample for 2 ½ hours at 105°C.
8. Weigh the crucible after drying and cooling in desiccator.
9. Weigh accurately 0.4000 g of the sample in a 250 ml glass beaker.
10. Weigh two samples for double determination. Deviation between double determinations must max. be 1.5% absolute. If deviation exceeds 1.5% the test must be repeated.
11. Wet the pectin with approx. 2 ml 100% IPA and add approx. 100 ml carbon di-oxide-free, deionized water while stirring on a magnetic stirrer.

(Chloride test on ash-free and moisture-free basis: Transfer approximately 10 ml filtrate to a test tube, add approximately 3 ml 3 N $HNO_3$, and add a few drops of $AgNO_3$. The filtrate will be chloride-free if the solution is clear, otherwise there will be a precipitation of silver chloride.)

The sample is now ready for titration, either by means of an indicator or by using a pH-meter/autoburette.

Procedure - Determination of % DE only
(Acid alcohol: 100 ml 60% IPA + 5 ml HCl fuming 37%):

1. Weigh 2.00 g pectin in a 250 ml glass beaker.
2. Add 100 ml acid alcohol and stir on a magnetic stirrer for 10 minutes.
3. Filtrate through a Büchner funnel with filter paper.

4. Rinse the beaker with 90 ml acid alcohol.
5. Wash with 1000 ml 60% IPA.
6. Wash with approximately 30 ml 100% IPA.
7. Dry the sample for approximately 15 minutes on Büchner funnel with vacuum suction.
8. Weigh approximately 0.40 g of the sample in a 250 ml glass beaker.
9. Weigh two samples for double determination. Deviation between double determinations must max. be 1.5% absolute. If deviation exceeds 1.5% the test must be repeated.
10. Wet the pectin with approximately 2 ml 100% IPA and add approx. 100 ml de-ionized water while stirring on a magnetic stirrer.

The sample is now ready for titration, either by means of an indicator or by using a pH-meter/autoburette.
Note: It is very important that samples with % DE < 10% are titrated very slowly, as the sample will only dissolve slowly during titration.
Titration using indicator:

1. Add 5 drops of phenolphtalein indicator and titrate with 0.1 N NaOH until change of color (record it as $V_1$ titer).
2. Add 20.00 ml 0.5 N NaOH while stirring. Let stand for exactly 15 min. When standing the sample must be covered with foil.
3. Add 20.00 ml 0.5 N HCl while stirring and stir until the color disappears.
4. Add 3 drops of phenolphtalein and titrate with 0,1 N NaOH until change of color (record it as $V_2$ titer).

Blind test (Double determination is carried out):

1. Add 5 drops phenolphtalein to 100 ml carbon dioxide-free or dionized water (same type as used for the sample), and titrate in a 250 ml glass beaker with 0.1 N NaOH until change of color (1-2 drops).
2. Add 20.00 ml 0.5 N NaOH and let the sample stand untouched for exactly 15 minutes. When standing the sample must be covered with foil.
3. Add 20.00 ml 0.5 N HCl and 3 drops phenolphtalein, and titrate until change of color with 0.1 N NaOH (record it as $B_1$). Maximum amount allowed for titration is 1 ml 0.1 N NaOH. If titrating with more than 1 ml, 0.5 N HCl must be diluted with a small amount of deionized water. If the sample has shown change of color on addition of 0.5 N HCl, 0.5 N NaOH must be diluted with a small amount of carbon dioxide-free water. Maximum allowed dilution with water is such that the solutions are between 0.52 and 0.48 N.

Titration using pH-meter/Autoburette:

Using Autoburette type ABU 80 the following settings may be applied:

| Sample with | % DE<10 | Blind test |
|---|---|---|
| Proportional band | 0.5 | 5 |
| Delay sec. | 50 | 5 |
| Speed - V1 | 10 | 5 |
| Speed - V2 | 15 | 5 |

1. Titrate with 0.1 N NaOH to pH 8.5 (record the result as $V_1$ titer).
2. Add 20.00 ml 0.5 N NaOH while stirring, and let the sample stand without stirring for exactly 15 minutes. When standing the sample must be covered with foil.
3. Add 20.00 ml 0.5 N HCl while stirring and stir until pH is constant.
4. Subsequently, titrate with 0.1 N NaOH to pH 8.5 (record the result as $V_2$ titer).

Blind test (Double determination is carried out):

1. Titrate 100 ml carbon dioxide-free or deionized (same type as used for the sample) water to pH 8.5 with 0.1 N NaOH (1-2 drops).
2. Add 20.00 ml 0.5 N NaOH while stirring and let the blind test sample stand without stirring for exactly 15 min. When standing the sample must be covered with foil.

3. Add 20.00 ml 0.5 N HCl while stirring, and stir until pH is constant.
4. Titrate to pH 8.5 with 0.1 N NaOH (record it as $B_1$). Maximum amount allowed for titration is 1 ml 0.1 N NaOH. If titrating with more than 1 ml, 0.5 N HCl must be diluted with a small amount of deionized water. If pH does not fall to below 8.5 on addition of 0.5 N HCl, 0.5 N NaOH must be diluted with a small amount of carbon dioxide-free water. Maximum allowed dilution with water is such that the dilutions are between 0.52 and 0.48 N.

Calculation:

$$V_t = V_1 + (V_2 - B_1)$$

$$\%\ \text{DE (Degree of Esterification)} = \{(V_2 - B_1) \times 100\} / V_t$$

$$\%\ \text{DFA (Degree of Free Acid)} = 100 - \%\ \text{DE}$$

$$\%\ \text{GA* (Degree of Galacturonic acid)} = (194.1 \times V_t \times N \times 100) / 400$$

194.1: Molecular weight for GA
N: Corrected normality for 0.1 N NaOH used for titration (e.g. 0.1002 N)
400: weight in mg of washed and dried sample for titration

$$\%\ \text{Pure pectin} = \{(\text{acid washed, dried amount of pectin}) \times 100\} / (\text{weighed amount of pectin})$$

h) Determination of pH-drop

1. 1 g. pectin was dissolved in 100 g. deionized water at 70°C and at 20°C.
2. The solution was placed in a thermostatically controlled water bath and continuously stirred.
3. 0.1 M NaOH was added to a pH of between 9 and 10.
4. The pH was recorded as a function of time

i) Determination of titration curves

1. 2 g. pectin was dissolved in 200 g. deionized water at 70°C and at 20°C.
2. The solution was placed in a thermostatically controlled water bath at 25°C and continuously stirred.
3. 0.1 M NaOH was added to the solution and pH recorded as a function of added 0.1 M NaOH.

j) Propylene glycol alginate - Quantitative determination of the ester groups is carried out by the saponification method described on pages 169-172 of "Quantitative organic analysis via functional groups", 4th Edition, John Wiley and Sons Publication.

1. Kelcoloid O manufactured by ISP Technologies, Inc. Esterification: High - about 85%.
2. Manucol Ester ER/K manufactured by ISP Technologies, Inc. Esterification: High - about 80%.
3. Kelcoloid HVF manufactured by ISP Technologies, Inc. Esterification: Medium - about 55%

k) Preparation of lotion and pH-drop in lotion

Lotions were prepared according to the composition:

Table 2.1.1: Composition of lotion

| Ingredient | grams | % | Comment |
|---|---|---|---|
| Isopropyl Palmitate | 59 | 18,11 | Waglinol 6016; manufactured by Industrail Quimica Lasem SA; Spain |
| Emulsifier | 20 | 6,14 | Emulium Delta; manufactured by Gattefossé, France |
| Sodium benzoate | 0,22 | 0,07 | Analytical grade; manufactured by Merck, Germany, 0.09% in water |
| Potassium sorbate | 0,15 | 0,05 | Analytical grade; manufactured by Fluka, Switzerland, 0.06% in water |
| Pectin | 2,44 | 0,75 | 1.00% in water, DE=81.7% |
| Distilled water | 244 | 74,89 | |
| Total | 325,81 | 100 | pH of lotion: 3 - 4 |

Since the pH is low, the lotion can be preserved with conventional food-grade preservatives.

Method 1:

1. Palmitate and emulsifier were mixed and heated to 75°C in order to melt the emulsifier.
2. Pectin and preservatives were dispersed in distilled water and heated to 75°C.
3. The hot oil phase was added to the hot water phase while stirring on magnetic stirrer.
4. The mix was cooled to about 30°C on cooling bath while stirring and fill into appropriate container.

Method 2:

1. Palmitate and emulsifier were mixed and heated to 75°C in order to melt the emulsifier.
2. Pectin was dispersed into the hot melt. Pectin is insoluble in the oil phase and consequently easy to disperse therein without formation of lumps.
3. Preservatives were dissolved in distilled water and the solution was heated to 75°C.
4. The hot oil phase was added to the hot water phase while stirring on magnetic stirrer.
5. The mix was cooled to about 30°C on cooling bath while stirring and fill into appropriate container.

1) Rinsing test - Note: This test is indicative, only. It is not possible to read the pH accurately.

1. A piece of cotton was cut to fit into a petri dish.
2. The cotton piece was soaked in a pectin solution in distilled water and stirred on magnetic stirrer for about 5 minutes.
3. The wet cloth was hand-pressed and placed in a petri dish.
4. The cloth was dried over night in an oven at 50°C.
5. The dried cloth was wetted with 2 ml 0.001 M NaOH.
6. A piece of indicator paper (pH in the range 1 - 11) was placed on the cloth.
7. The color change of the indicator paper over time was recorded.

**Examples**

The following examples are non-limiting.

Example 1: Effect of molecular weight

Five samples of different molecular weight, but with similar DE made from dried lemon peel were titrated and the pH drop over time recorded for samples dissolved at 70°C and 20°C, respectively. The pH drop was measured at 30 - 32°C. Titration was done using 0.1008 M NaOH. The comment "unstable" refers to the pH-meter, which at high pH values showed an unstable reading.

1. 97CP001-39-0: Mw=123.000; DE=71.4%

Table 1.1: Titration and pH drop of pectin with molecular weight 123.000

| ml NaOH | pH | Comment | Time, minutes, 70°C | pH | Time, minutes, 20°C | pH |
|---|---|---|---|---|---|---|
| 0 | 3,12 | | 0 | 9,83 | 0 | 9,72 |
| 1 | 3,19 | | 1 | 9,47 | 1 | 9,4 |
| 2 | 3,26 | | 2 | 9,27 | 2 | 9,21 |
| 3 | 3,33 | 3 | 3 | 9,13 | 3 | 9,08 |
| 4 | 3,41 | | 4 | 9,03 | 4 | 8,98 |
| 5 | 3,48 | 5 | 5 | 8,94 | 5 | 8,9 |
| 6 | 3,56 | | 18 | 8,39 | 6 | 8,82 |
| 7 | 3,63 | | 54 | 7,66 | 19 | 8,29 |
| 8 | 3,71 | | 72 | 7,38 | 30 | 8,08 |
| 9 | 3,8 | | 102 | 7,16 | 62 | 7,44 |
| 10 | 3,91 | | 175 | 6,86 | 97 | 7,15 |
| 11 | 4,02 | | 1149 | 6,15 | 157 | 6,92 |
| 12 | 4,15 | | 1194 | 6,12 | 193 | 6,85 |
| 13 | 4,29 | | | | | |
| 14 | 4,48 | | | | | |
| 15 | 4,72 | | | | | |
| 16 | 5,13 | | | | | |
| 17 | 6,84 | | | | | |
| 17,5 | 8,52 | Unstable | | | | |
| 18 | 8,95 | Unstable | | | | |

2. 97CP001-39-1: Mw=108.500; DE=71.4%

Table 1.2: Titration and pH drop of pectin with molecular weight 108.500

| ml NAOH | pH | Comment | Time, minutes, 70°C | pH | Time, minutes, 20°C | pH |
|---|---|---|---|---|---|---|
| 0 | 3,12 | | 0 | 9,7 | 0 | 9,64 |
| 1 | 3,17 | | 1 | 9,44 | 1 | 9,35 |
| 2 | 3,24 | | 2 | 9,28 | 2 | 9,16 |
| 3 | 3,3 | | 3 | 9,13 | 3 | 9,02 |
| 4 | 3,37 | | 4 | 9,04 | 4 | 8,91 |

(continued)

| ml NAOH | pH | Comment | Time, minutes, 70°C | pH | Time, minutes, 20°C | pH |
|---|---|---|---|---|---|---|
| 5 | 3,44 | | 5 | 8,93 | 5 | 8,83 |
| 6 | 3,52 | | 8 | 8,66 | 15 | 8,3 |
| 7 | 3,59 | | 22 | 7,98 | 23 | 8,06 |
| 8 | 3,68 | | 61 | 7,42 | 30 | 7,81 |
| 9 | 3,77 | | 101 | 7,02 | 43 | 7,48 |
| 10 | 3,87 | | 158 | 6,88 | 56 | 7,32 |
| 11 | 3,97 | | 188 | 6,84 | 69 | 7,22 |
| 12 | 4,09 | | 1171 | 6,09 | 103 | 6,99 |
| 13 | 4,22 | | 1188 | 6,05 | 159 | 6,82 |
| 14 | 4,39 | | | | 210 | 6,74 |
| 15 | 4,6 | | | | | |
| 16 | 4,91 | | | | | |
| 17 | 5,55 | | | | | |
| 17,5 | 7,02 | Unstable | | | | |
| 18 | 8,71 | Unstable | | | | |

3. 97CP001-39-2: Mw=95.000; DE=72.3%

Table 1.3: Titration and pH drop of pectin with molecular weight 95.000

| ml NaOH | pH | Comment | Time, minutes, 70°C | pH | Time, minutes, 20°C | pH |
|---|---|---|---|---|---|---|
| 0 | 3,03 | | 0 | 9,9 | 0 | 9,57 |
| 1 | 3,1 | | 1 | 9,55 | 1 | 9,33 |
| 2 | 3,15 | | 2 | 9,33 | 2 | 9,18 |
| 3 | 3,22 | | 3 | 9,22 | 3 | 9,06 |
| 4 | 3,29 | | 4 | 9,13 | 4 | 8,97 |
| 5 | 3,36 | | 5 | 9,04 | 5 | 8,89 |
| 6 | 3,43 | | 6 | 8,98 | 6 | 8,82 |
| 7 | 3,51 | | 13 | 8,61 | 41 | 7,52 |
| 8 | 3,59 | | 25 | 8,05 | 49 | 7,38 |
| 9 | 3,67 | | 31 | 7,87 | 66 | 7,23 |
| 10 | 3,77 | | 49 | 7,51 | 78 | 7,16 |
| 11 | 3,84 | | 66 | 7,34 | 100 | 7,11 |
| 12 | 3,95 | | 104 | 7,04 | 141 | 6,96 |
| 13 | 4,08 | | 128 | 6,95 | 162 | 6,92 |
| 14 | 4,2 | | 159 | 6,89 | 172 | 6,92 |
| 15 | 4,35 | | 1488 | 6,2 | | |
| 16 | 4,56 | | | | | |

(continued)

| ml NaOH | pH | Comment | Time, minutes, 70°C | pH | Time, minutes, 20°C | pH |
|---|---|---|---|---|---|---|
| 17 | 4,84 | | | | | |
| 18 | 5,37 | | | | | |
| 18,5 | 6,12 | | | | | |
| 19 | 8,32 | Unstable | | | | |
| 19,5 | 8,94 | Unstable | | | | |

4. 97CP001-39-4: Mw=71.500; DE=71.6%

Table 1.4: Titration and pH drop of pectin with molecular weight 71.500

| ml NaOH | pH | Comment | Time, minutes, 70°C | pH | Time, minutes, 20°C | pH |
|---|---|---|---|---|---|---|
| 0 | 3,06 | | 0 | 9,83 | 0 | 9,59 |
| 1 | 3,12 | | 1 | 9,38 | 1 | 9,3 |
| 2 | 3,18 | | 2 | 9,13 | 2 | 9,1 |
| 3 | 3,25 | | 3 | 8,95 | 3 | 8,98 |
| 4 | 3,32 | | 4 | 8,84 | 4 | 8,88 |
| 5 | 3,38 | | 5 | 8,73 | 5 | 8,79 |
| 6 | 3,45 | | 24 | 8,18 | 11 | 8,44 |
| 7 | 3,53 | | 30 | 7,95 | 20 | 8,09 |
| 8 | 3,61 | | 56 | 7,37 | 37 | 7,56 |
| 9 | 3,69 | | 87 | 7,07 | 55 | 7,29 |
| 10 | 3,78 | | 115 | 6,93 | 84 | 7,06 |
| 11 | 3,87 | | 163 | 6,82 | 164 | 6,86 |
| 12 | 3,98 | | 225 | 6,74 | 176 | 6,84 |
| 13 | 4,1 | 310 | 310 | 6,65 | | |
| 14 | 4,24 | | 1216 | 6,09 | | |
| 15 | 4,4 | | 1252 | 6,04 | | |
| 16 | 4,61 | | | | | |
| 17 | 4,91 | | | | | |
| 17,5 | 5,14 | | | | | |
| 18 | 5,52 | | | | | |
| 18,5 | 6,77 | Unstable | | | | |
| 19 | 8,63 | Unstable | | | | |

5. 97CP001-39-5: Mw=41.500; DE=73%

Table 1.5: Titration and pH drop of pectin with molecular weight 41.500

| ml NaOH | pH | Comment | Time, minutes, 70°C | pH | Time, minutes, 20°C | pH |
|---|---|---|---|---|---|---|
| 0 | 3,04 | | 0 | 9,83 | 0 | 9,53 |
| 1 | 3,09 | | 1 | 9,36 | 1 | 9,22 |
| 2 | 3,15 | | 2 | 9,1 | 2 | 9,05 |
| 3 | 3,21 | | 3 | 8,93 | 3 | 8,9 |
| 4 | 3,28 | | 4 | 8,81 | 4 | 8,78 |
| 5 | 3,34 | | 5 | 8,7 | 5 | 8,68 |
| 6 | 3,41 | | 10 | 8,34 | 6 | 8,61 |
| 7 | 3,48 | | 19 | 7,93 | 20 | 7,94 |
| 8 | 3,56 | | 29 | 7,64 | 26 | 7,77 |
| 9 | 3,64 | | 45 | 7,34 | 55 | 7,22 |
| 10 | 3,73 | | 67 | 7,13 | 76 | 7,11 |
| 11 | 3,81 | | 129 | 6,9 | 122 | 6,93 |
| 12 | 3,91 | | 199 | 6,8 | 159 | 6,87 |
| 13 | 4,02 | | 1173 | 5,88 | 261 | 6,73 |
| 14 | 4,15 | | 1193 | 5,85 | | |
| 15 | 4,29 | | | | | |
| 16 | 4,46 | | | | | |
| 17 | 4,7 | | | | | |
| 18 | 5,06 | | | | | |
| 18,5 | 5,39 | | | | | |
| 19 | 6,22 | Unstable | | | | |
| 19,5 | 8,47 | Unstable | | | | |
| 20 | 9,04 | Unstable | | | | |

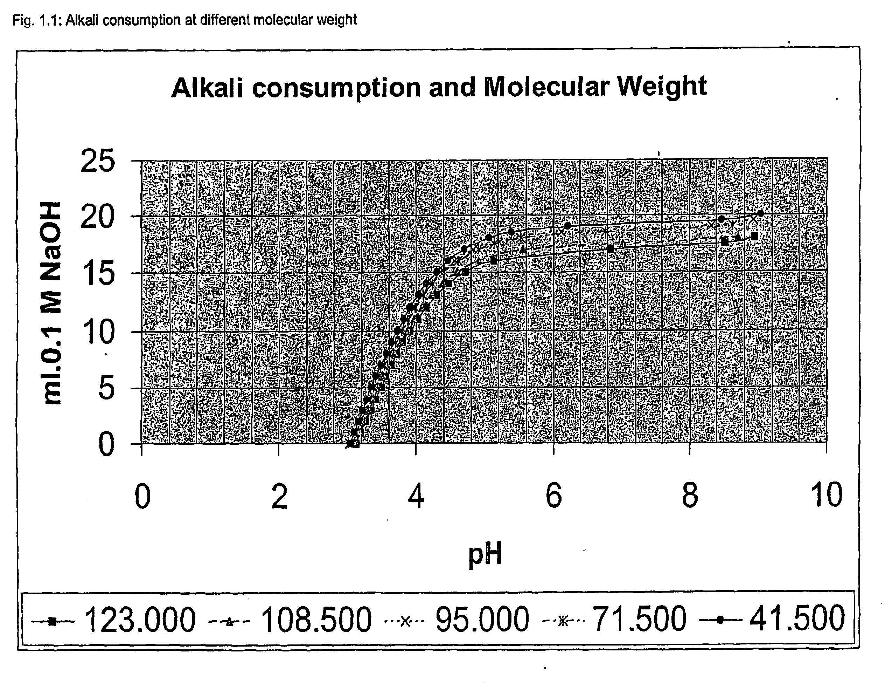

Fig. 1.1 shows that the molecular weight of pectin has no influence on the alkali consumption.

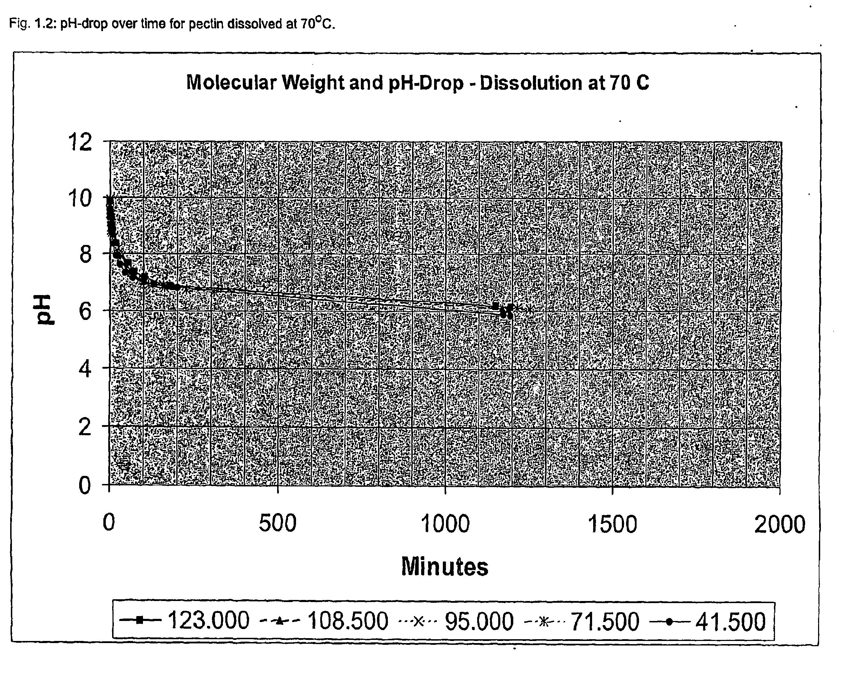

The data in fig. 1.2 do not suggest a change in the pH-drop resulting from a change in molecular weight. In practice, this means that a pH controlling preparation made from pectin can be made thick (high molecular weight) or thin (low molecular weight) or basically with any viscosity between the two extremes. In addition, if the alkali consumption is to be increased, a low molecular weight pectin preparation makes it possible to increase the concentration of pectin without making the alkali consuming preparation too viscous.

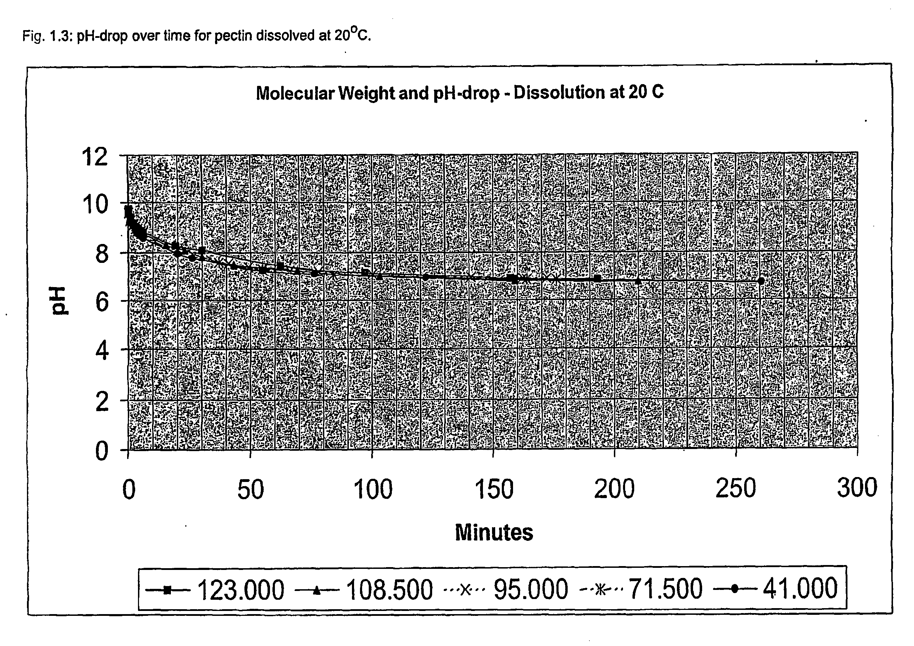

Fig. 1.3 shows that dissolution temperature does not change the drop in pH. Thus, irrespective of the molecular weight, pectin preparation for controlling pH can be made either hot or cold.

Example 2: Effect of Degree of Esterification

Eight samples were prepared with different degree of esterification ranging from about 9 to 93%. The samples were made from dried lemon peel. All were titrated and the pH drop over time recorded for samples dissolved at 70°C and 20°C, respectively. The pH drop was measured at 30 - 32°C. Titration was done using 0.1008 M NaOH. The comment "unstable" refers to the pH-meter, which at high pH values showed an unstable reading.

1. Sample with DE=9.6%

Table 2.1: Titration and pH drop of pectin with DE=9.6%

| ml NAOH | pH | Comment | Time, minutes, 70°C | pH | Time, minutes, 20°C | pH |
|---|---|---|---|---|---|---|
| 0 | 4,07 | | 0 | 9,76 | 0 | 9,62 |
| 1 | 4,12 | | 1 | 9,69 | 1 | 9,57 |
| 2 | 4,16 | | 2 | 9,64 | 2 | 9,54 |
| 3 | 4,2 | 3 | 3 | 9,59 | 3 | 9,5 |
| 4 | 4,24 | | 4 | 9,57 | 4 | 9,48 |
| 5 | 4,28 | | 12 | 9,31 | 5 | 9,45 |
| 6 | 4,33 | | 32 | 9,06 | 7 | 9,41 |
| 7 | 4,37 | | 74 | 8,56 | 12 | 9,05 |
| 8 | 4,42 | | 112 | 8,15 | 22 | 8,92 |
| 9 | 4,47 | | 1479 | 7,27 | 49 | 8,76 |
| 10 | 4,52 | 4093 | 4093 | 6,26 | 62 | 8,56 |
| 11 | 4,57 | | | | 122 | 7,98 |
| 12 | 4,64 | | | | 182 | 7,6 |
| 13 | 4,7 | | | 242 | 242 | 7,47 |
| 14 | 4,77 | | | | 302 | 7,37 |
| 15 | 4,86 | | | | 449 | 7,32 |
| 16 | 4,96 | | | | 1382 | 7,21 |
| 17 | 5,08 | | | | 1412 | 7,18 |
| 18 | 5,23 | | | | | |
| 19 | 5,45 | | | | | |
| 20 | 5,85 | | | | | |
| 21 | 8,17 | Unstable | | | | |

2. Sample with DE=34.4%

Table 2.2: Titration and pH drop of pectin with DE=34.4%

| ml NaOH | pH | Comment | Time, minutes, 70°C | pH | Time, minutes, 20°C | pH |
|---|---|---|---|---|---|---|
| 0 | 3,22 | | 0 | 9,97 | 0 | 9,7 |
| 1 | 3,27 | | 1 | 9,74 | 1 | 9,54 |
| 2 | 3,3 | | 2 | 9,59 | 2 | 9,43 |
| 3 | 3,33 | | 3 | 9,48 | 3 | 9,33 |
| 4 | 3,36 | | 4 | 9,37 | 4 | 9,25 |
| 5 | 3,39 | | 5 | 9,28 | 5 | 9,18 |
| 6 | 3,42 | | 35 | 8,01 | 12 | 8,78 |
| 7 | 3,45 | | 67 | 7,59 | 26 | 8,15 |
| 8 | 3,48 | | 110 | 7,33 | 58 | 7,65 |
| 9 | 3,51 | | 151 | 7,19 | 88 | 7,41 |

(continued)

| ml NaOH | pH | Comment | Time, minutes, 70°C | pH | Time, minutes, 20°C | pH |
|---|---|---|---|---|---|---|
| 10 | 3,55 | | 1483 | 6,54 | 189 | 7,1 |
| 11 | 3,58 | | | | | |
| 12 | 3,62 | | | | | |
| 13 | 3,65 | | | | | |
| 14 | 3,69 | | | | | |
| 15 | 3,74 | | | | | |
| 16 | 3,77 | | | | | |
| 17 | 3,82 | | | | | |
| 18 | 3,86 | | | | | |
| 19 | 3,9 | | | | | |
| 20 | 3,94 | | | | | |
| 21 | 3,98 | | | | | |
| 22 | 4,03 | | | | | |
| 23 | 4,08 | | | | | |
| 24 | 4,13 | | | | | |
| 25 | 4,17 | | | | | |
| 26 | 4,23 | | | | | |
| 27 | 4,28 | | | | | |
| 28 | 4,34 | | | | | |
| 29 | 4,4 | | | | | |
| 30 | 4,47 | | | | | |
| 31 | 4,54 | | | | | |
| 33 | 4,72 | | | | | |
| 35 | 4,97 | | | | | |
| 36 | 5,16 | | | | | |
| 37 | 5,45 | | | | | |
| 38 | 6,2 | | | | | |
| 39 | 9,76 | Unstable | | | | |

3. Sample with DE=71%

Table 2.3: Titration and pH drop of pectin with DE=71%

| ml NaOH | pH | Comment | Time, minutes, 70°C | pH | Time, minutes, 20°C | pH |
|---|---|---|---|---|---|---|
| 0 | 3,11 | | 0 | 10,2 1 | 0 | 9,73 |
| 0,2 | 3,12 | | 0,5 | 9,85 | 1 | 9,24 |
| 0,42 | 3,14 | | 1 | 9,65 | 2 | 8,92 |
| 0,6 | 3,15 | 2 | 2 | 9,35 | 3 | 8,68 |

(continued)

| ml NaOH | pH | Comment | Time, minutes, 70°C | pH | Time, minutes, 20°C | pH |
|---|---|---|---|---|---|---|
| 0,84 | 3,17 | 3 | 3 | 9,1 | 4 | 8,48 |
| 1,2 | 3,2 | | 8 | 8,39 | 9 | 7,88 |
| 1,6 | 3,23 | | 10 | 8,21 | 14 | 7,56 |
| 2,08 | 3,27 | 20 | 20 | 7,73 | 23 | 7,23 |
| 2,4 | 3,29 | | 31 | 7,5 | 33 | 7,07 |
| 3 | 3,34 | | 45 | 7,3 | 44 | 6,96 |
| 3,4 | 3,37 | | 75 | 7,12 | 48 | 6,94 |
| 4 | 3,42 | | 115 | 7 | | |
| 4,8 | 3,49 | | 150 | 6,91 | | |
| 5,68 | 3,56 | | 190 | 6,86 | | |
| 6,02 | 3,59 | | 260 | 6,85 | | |
| 6,6 | 3,64 | | 285 | 6,82 | | |
| 7,6 | 3,73 | | 320 | 6,78 | | |
| 8 | 3,76 | | 360 | 6,75 | | |
| 9 | 3,86 | | 390 | 6,73 | | |
| 10 | 3,97 | | | | | |
| 10,4 | 4 | | | | | |
| 11 | 4,07 | | | | | |
| 12 | 4,2 | | | | | |
| 13 | 4,34 | | | | | |
| 14 | 4,52 | | | | | |
| 15 | 4,73 | | | | | |
| 16 | 5,08 | | | | | |
| 16,6 | 5,43 | | | | | |
| 17 | 5,95 | | | | | |
| 17,4 | 8,12 | Unstable | | | | |
| 17,6 | 9 | Unstable | | | | |

4. Sample with DE=93.4%

Table 2.4: Titration and pH drop of pectin with DE=93.4%

| ml NaOH | pH | Comment | Time, minutes, 70°C | pH | Time, minutes, 20°C | pH |
|---|---|---|---|---|---|---|
| 0 | 3,26 | | 0 | 9,5 | 0 | 9,29 |
| 1 | 3,43 | | 1 | 8,89 | 1 | 8,14 |
| 2 | 3,65 | | 2 | 8,14 | 2 | 7,7 |
| 3 | 3,98 | 3 | 3 | 7,77 | 3 | 7,49 |
| 4 | 4,54 | 4 | 4 | 7,58 | 4 | 7,33 |

(continued)

| ml NaOH | pH | Comment | Time, minutes, 70°C | pH | Time, minutes, 20°C | pH |
|---|---|---|---|---|---|---|
| 5 | 8,74 | Unstable | 5 | 7,45 | 5 | 7,21 |
| | | | 11 | 7,04 | 8 | 7 |
| | | | 15 | 6,9 | 13 | 6,81 |
| | | 20 | 20 | 6,79 | 23 | 6,61 |
| | | 25 | 25 | 6,7 | 33 | 6,51 |
| | | | 30 | 6,62 | 1004 | 5,37 |
| | | 38 | 38 | 6,52 | 1018 | 5,3 |

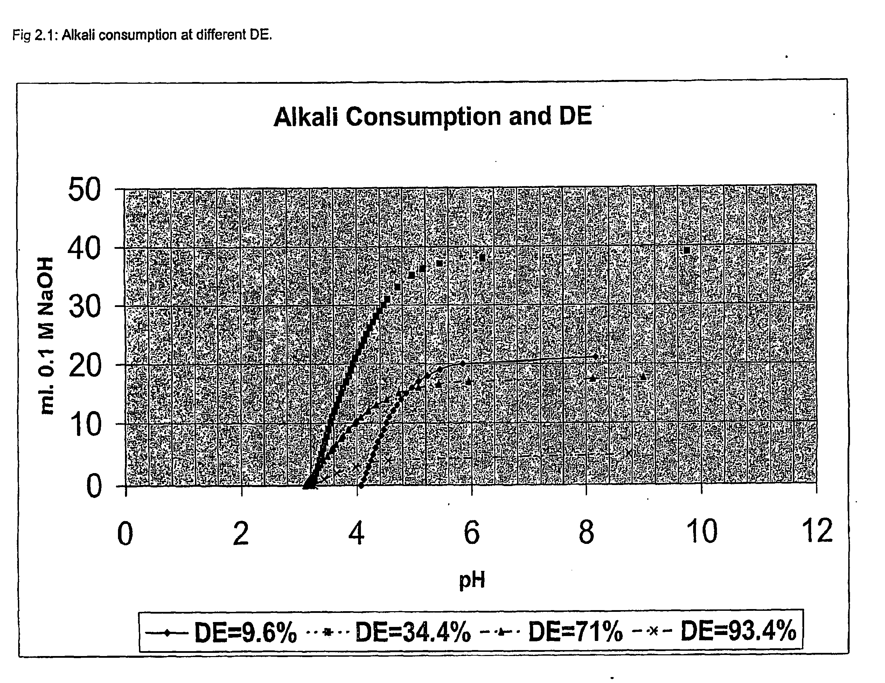

Fig 2.1 shows that one pectin is characterized by a higher starting pH than the rest. Conventionally, pectin is neutralized with an alkali metal base to a pH in the range 3 - 4 or even higher. This is mainly in order to preserve the pectin, but it also has an impact on the solubility of the pectin. However, if one moves the curve for DE=9.6% upwards to connect with the other curves, the picture becomes clear: With increasing DE and consequently decreasing galacturonic acid, the pectin can consume less alkali. Thus, if pectin is used to neutralize alkali, the degree of esterification and the starting pH should be as low as possible.

To further elaborate on this point, I define buffer capacity as ml. 0.1 M NaOH required to increase the pH by 1 pH unit, calculated from the part of the titration curve, which is steepest.

Thus, the approximate buffer capacities as calculated from fig. 2.1 are:

- DE=9.6% and DE=34.4%: Buffer capacity about 26
- DE=71%: Buffer capacity about 12
- DE=93.4%: Buffer capacity less than 6

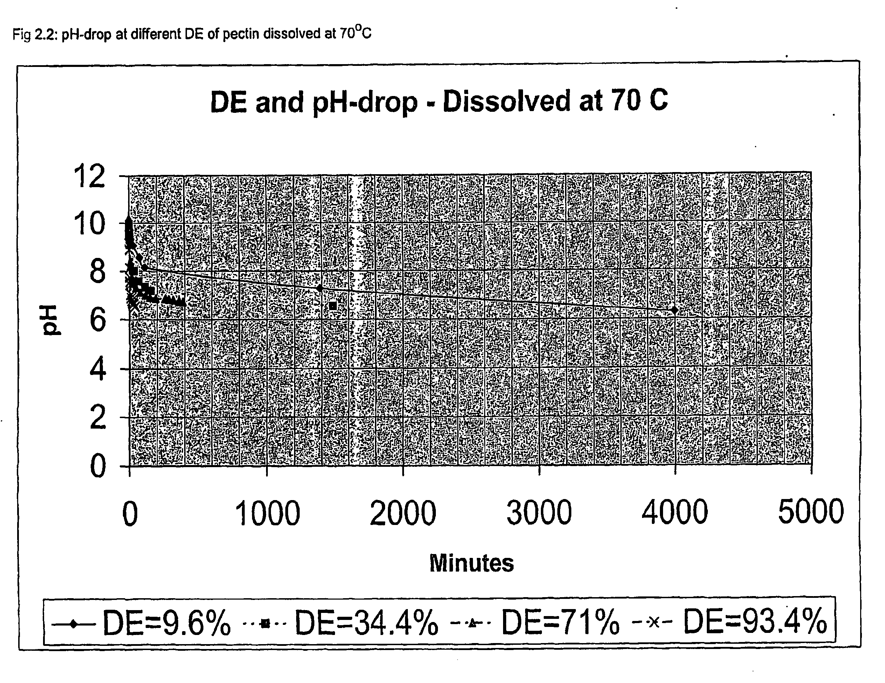

Fig. 2.2 show a dramatic increase in the pH-drop as the degree of esterification is increased.

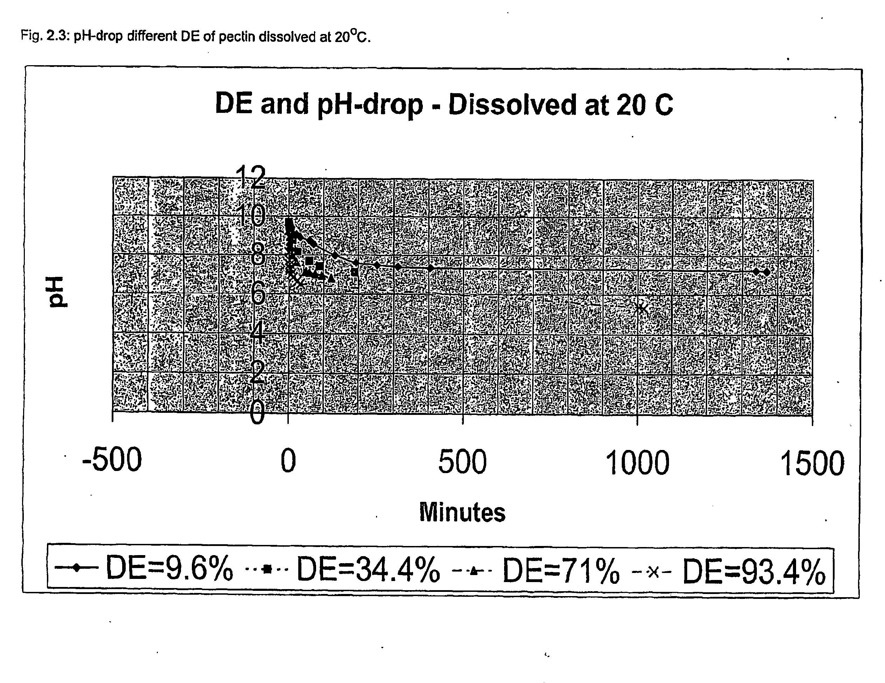

Fig. 2.3 shows the same dramatic influence of DE even when the pectin is dissolved at 20°C. The figure shows that at the high DE, the pH is eventually decreased below 5.5.

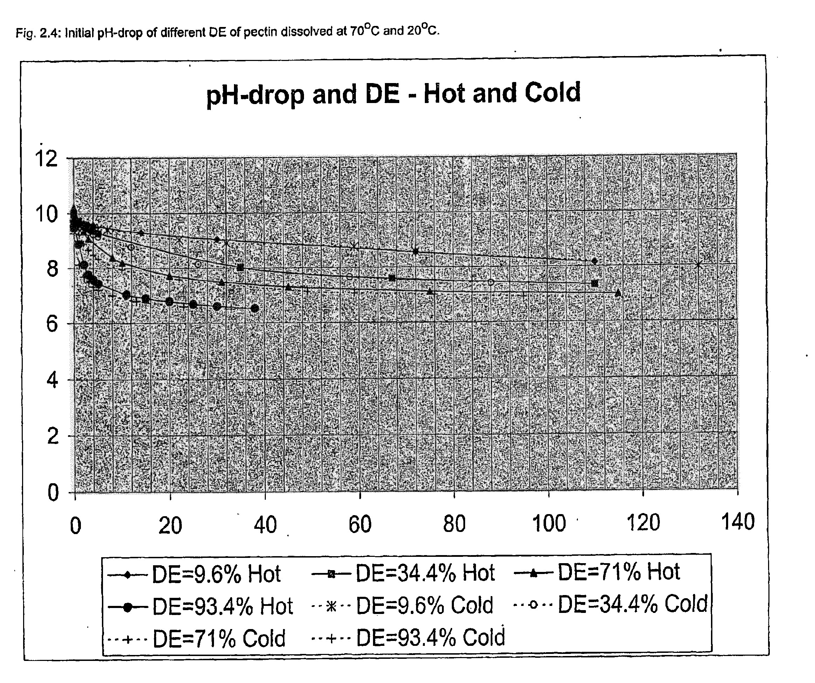

These results are compiled in fig. 2.4, in which the pH drop has been followed for the first up to about 130 minutes. It is evident that the pH-drop occurs to the same extent whether the pectin solution is made hot or cold.

For DE=93.4%, time to reach pH=8 is 2 minutes, for DE=71% it takes 12 minutes, for DE=34.4% the time is 35 minutes and for DE=9.6% it takes 130 minutes. In order to reach pH=7, the difference is even bigger. Pectin with a DE=71 is about 9 times slower than pectin with DE=93.4, and pectin with DE lower than 71% are slower than a factor 10 compared to pectin with DE=93.4.

Thus, if one needs to obtain a rapid pH decrease as a result of alkali generation, pectin with as high a DE as possible is preferred. If, on the other hand, the need calls for slower reduction of pH, then a lower DE would be preferred. Selecting pectin of a specific DE makes it possible to reduce the pH at a specific rate.

Another aspect is to combine pectin preparations of different DE. For example, one might combine a low DE pectin and a high DE pectin to achieve initial alkali consumption or buffer capacity and to provide pH reduction, when the buffer capacity is used.

<u>Example 3: Effect of Methyl Ester Distribution</u>

Two samples were made from dried lemon peel. One was de-esterified with a bacterial pectin esterase, which results in a random distribution of the methyl ester groups. The other was de-esterified with a plant pectin esterase, which results in a block wise distribution of the methyl ester groups. The samples were made to similar DE. Both samples were titrated and the pH drop over time recorded for samples dissolved at 70°C and 20°C, respectively. The pH drop was measured at 30 - 32°C. Titration was done using 0.1008 M NaOH. The comment "unstable" refers to the pH-meter, which at high pH values showed an unstable reading.

1. Random methyl ester distribution - DE=57.3%

Table 3.1: Titration and pH drop of pectin with DE=57.3% randomly distributed

| ml NaOH | pH | Comment | Time, minutes, 70°C | pH | Time, minutes, 20°C | pH |
|---|---|---|---|---|---|---|
| 0 | 3,01 | | 0 | 10,1 | 0 | 9,94 |
| 1 | 3,05 | | 1 | 9,77 | 1 | 9,72 |
| 2 | 3,1 | | 2 | 9,62 | 2 | 9,63 |
| 3 | 3,15 | | 3 | 9,51 | 3 | 9,5 |
| 4 | 3,19 | | 4 | 9,42 | 4 | 9,43 |
| 5 | 3,24 | | 5 | 9,34 | 5 | 9,35 |
| 6 | 3,29 | | 12 | 9,03 | 6 | 9,3 |
| 7 | 3,33 | | 29 | 8,53 | 12 | 9,02 |
| 8 | 3,38 | | 68 | 7,92 | 26 | 8,58 |
| 9 | 3,43 | | 91 | 7,77 | 48 | 8,22 |
| 10 | 3,48 | | 116 | 7,46 | 83 | 7,74 |
| 11 | 3,52 | | 1095 | 6,4 | 103 | 7,59 |
| 12 | 3,58 | | 1110 | 6,34 | 135 | 7,36 |
| 13 | 3,63 | | | | 147 | 7,33 |
| 14 | 3,68 | | | | | |
| 15 | 3,74 | | | | | |
| 16 | 3,8 | | | | | |
| 17 | 3,86 | | | | | |
| 18 | 3,92 | | | | | |
| 19 | 3,99 | | | | | |
| 20 | 4,06 | | | | | |
| 21 | 4,13 | | | | | |
| 22 | 4,21 | | | | | |
| 23 | 4,3 | | | | | |
| 24 | 4,4 | | | | | |
| 25 | 4,52 | | | | | |
| 26 | 4,65 | | | | | |
| 27 | 4,83 | | | | | |
| 28 | 5,09 | | | | | |
| 29 | 5,61 | Unstable | | | | |
| 30 | 8,41 | Unstable | | | | |

2. Block wise methyl ester distribution - DE=57.7%

Table 3.2: Titration and pH drop of pectin with DE=57.7% and block wise distributed

| ml NaOH | pH | Comment | Time, minutes, 70°C | pH | Time, minutes, 20°C | pH |
|---|---|---|---|---|---|---|
| 0 | 3,42 | | 0 | 9,91 | 0 | 10,23 |

(continued)

| ml NaOH | pH | Comment | Time, minutes, 70°C | pH | Time, minutes, 20°C | pH |
|---|---|---|---|---|---|---|
| 1 | 3,49 | | 1 | 9,56 | 1 | 9,75 |
| 2 | 3,54 | | 2 | 9,32 | 2 | 9,44 |
| 3 | 3,6 | | 3 | 9,14 | 3 | 9,21 |
| 4 | 3,65 | | 4 | 9 | 4 | 9,03 |
| 5 | 3,71 | | 5 | 8,87 | 5 | 8,89 |
| 6 | 3,77 | | 14 | 8,07 | 8 | 8,57 |
| 7 | 3,83 | | 40 | 7,35 | 22 | 7,71 |
| 8 | 3,89 | | 61 | 7,15 | 35 | 7,38 |
| 9 | 3,96 | | 130 | 6,89 | 55 | 7,19 |
| 10 | 4,03 | | 190 | 6,81 | 76 | 7,08 |
| 11 | 4,11 | | 241 | 6,74 | 102 | 7,02 |
| 12 | 4,19 | | 1336 | 6,19 | 177 | 6,92 |
| 13 | 4,28 | | 1358 | 6,15 | 216 | 6,85 |
| 14 | 4,38 | | | | 269 | 6,8 |
| 15 | 4,5 | | | | | |
| 16 | 4,63 | | | | | |
| 17 | 4,79 | | | | | |
| 18 | 5,02 | | | | | |
| 19 | 5,42 | | | | | |
| 20 | 7,7 | Unstable | | | | |
| 21 | 10,0 1 | Unstable | | | | |

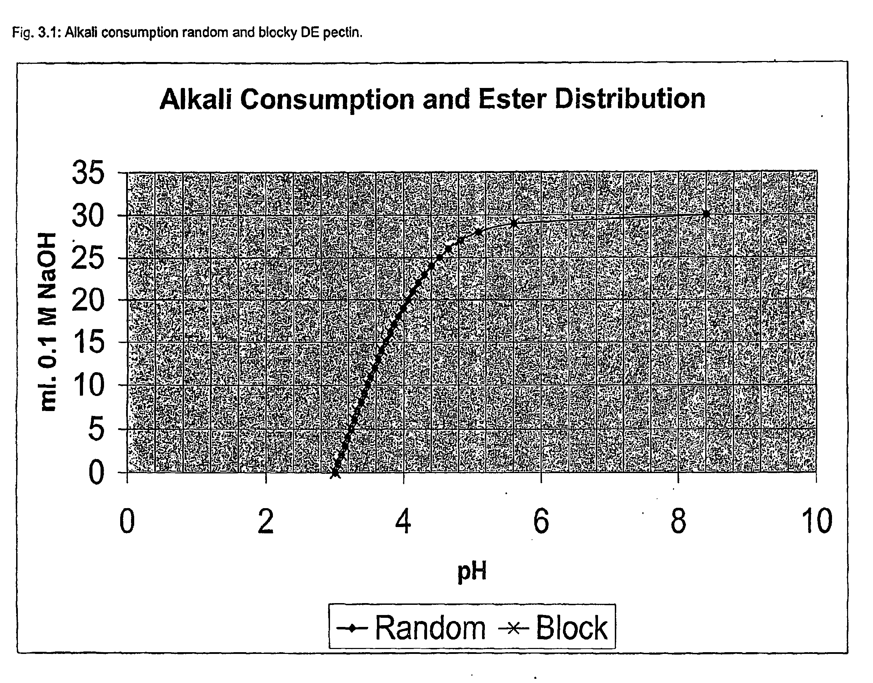

Fig. 3.1 shows that the distribution of methyl esters in pectin has no impact on the alkali consumption. The galacturonic acid drives the alkali consumption.

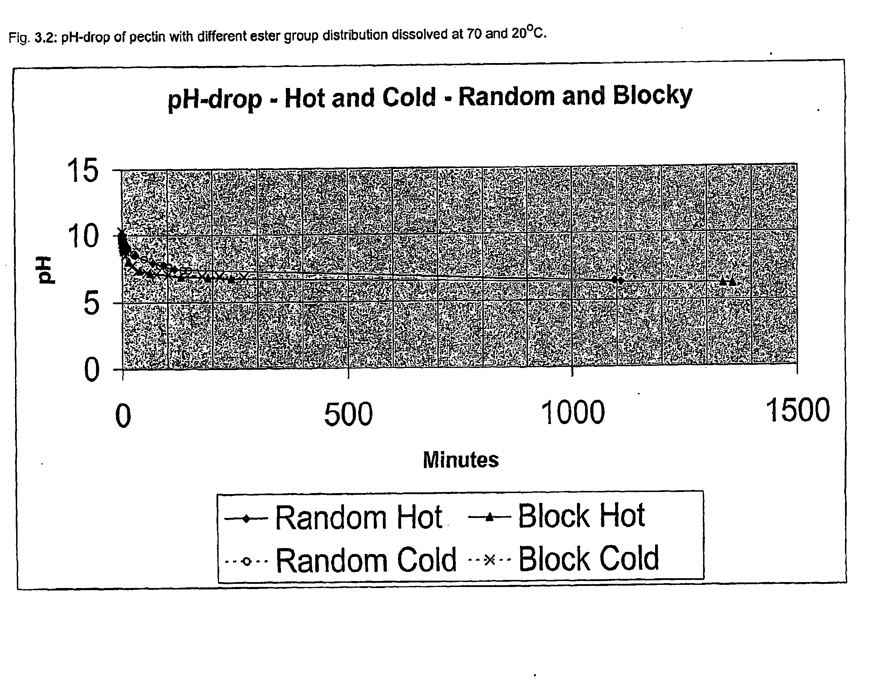

Fig. 3.2 indicates a difference in the rate of the pH-drop. It also shows, that identical pH-drop is achieved whether the pectin has been dissolved hot or cold.

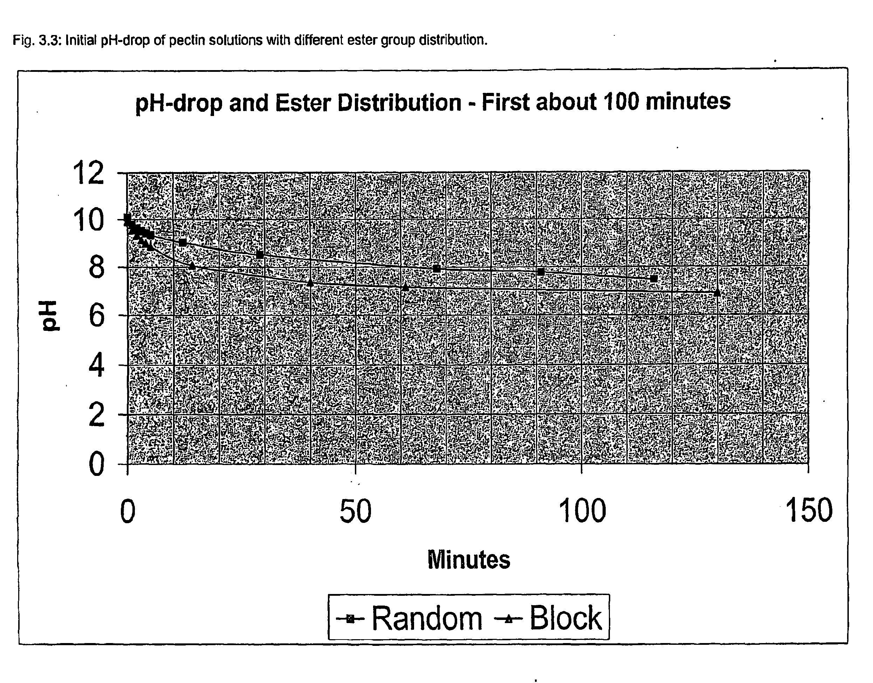

Fig. 3.3 shows the pH-drop in the first 120 - 130 minutes, and a random ester group distribution needs about 4 times longer to reach pH=8 compared to a blocky ester group distribution. Since the two pectin preparations have almost identical average DE, the faster pH-drop of a blocky ester distribution is explained by local concentration of ester groups. Thus, pectin with a blocky ester distribution will act as pectin with a higher average DE. In practice, this is important because one might treat a blocky pectin with polygalacturonase to increase the DE, which would constitute an easier way to make a high ester pectin than by using the process of re-methylation.

Example 4: Effect of temperature

The pH drop for one sample having DE=71% and made from dried lemon peel was recorded at four different temperatures. The sample was prepared by dissolving the pectin at 70°C and subsequently cooling the solution to the recording temperature. The temperature was maintained with a thermostatically controlled water bath.

1. Sample with DE=71%

Table 4.1: pH drop of pectin with DE=71% at various temperatures

| 25 - 27°C | | 30 - 32°C | | 35 - 37°C | | 45 - 47°C | |
|---|---|---|---|---|---|---|---|
| Time, minutes | pH | Time, minutes | pH | Time, minutes | pH | Time, minutes | pH |
| 0,2 | 10,4 | 0 | 10,2 1 | 0 | 10,0 8 | 0 | 10,0 1 |
| 0,5 | 10,2 1 | 0,5 | 9,85 | 1 | 9,41 | 1 | 9,15 |
| 1 | 9,85 | 1 | 9,65 | 2 | 9,01 | 2 | 8,59 |
| 2 | 9,52 | 2 | 9,35 | 3 | 8,73 | 3 | 8,26 |
| 3 | 9,26 | 3 | 9,1 | 10 | 7,81 | 4 | 8,04 |
| 5 | 8,89 | 8 | 8,39 | 23 | 7,4 | 9 | 7,49 |
| 10 | 8,33 | 10 | 8,21 | 35 | 7,23 | 16 | 7,15 |
| 20 | 7,75 | 20 | 7,73 | 143 | 6,75 | 21 | 7,02 |
| 30 | 7,47 | 31 | 7,5 | 203 | 6,64 | 44 | 6,78 |
| 45 | 7,24 | 45 | 7,3 | 263 | 6,57 | 61 | 6,68 |
| 60 | 7,11 | 75 | 7,12 | 326 | 6,5 | 76 | 6,62 |
| 122 | 6,91 | 115 | 7 | 378 | 6,45 | 121 | 6,5 |
| 181 | 6,85 | 150 | 6,91 | | | 154 | 6,43 |
| 240 | 6,79 | 190 | 6,86 | | | 202 | 6,34 |
| 291 | 6,77 | 260 | 6,85 | | | 250 | 6,27 |
| 347 | 6,74 | 285 | 6,82 | | | 325 | 6,17 |
| 1298 | 6,32 | 320 | 6,78 | | | 420 | 6,06 |
| 1428 | 6,31 | 360 | 6,75 | | | | |
| 1508 | 6,3 | 390 | 6,73 | | | | |
| 1553 | 6,28 | | | | | | |

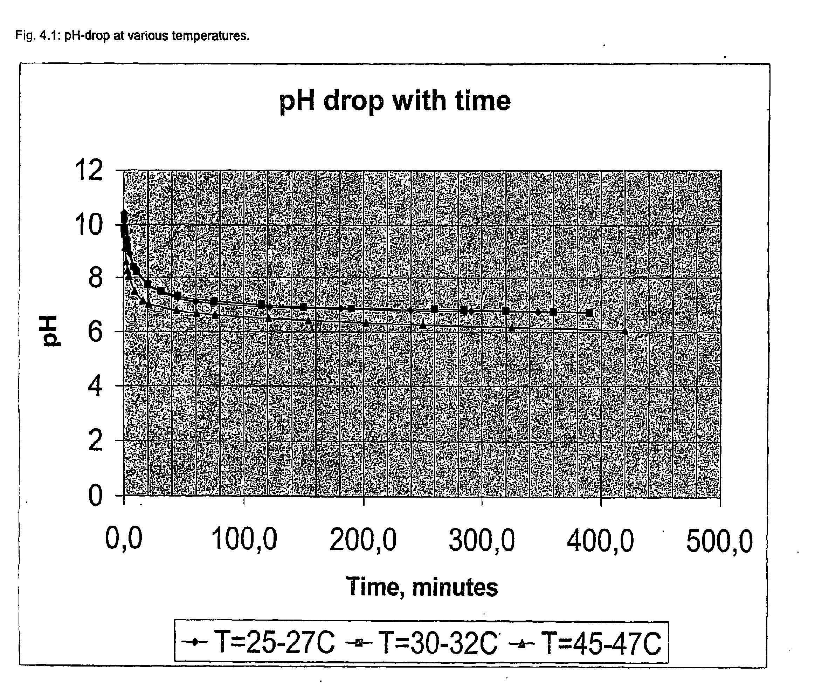

Fig. 4.1 shows that the rate of the pH-drop increases with increasing temperature. The rate is particularly increased as the temperature increases above about 30°C.

<u>Example 5: Effect of multiple additions of alkali</u>

The pH drop for one sample having DE=71% and made from dried lemon peel was recorded at a temperature of 25 - 27°C. First, the pH was raised to about 10 with 19 ml. 0.1 M NaOH. When the sample had reached a pH of 6 - 7, the pH was again raised to about 10. This required 1.1 ml. 0.1 M NaOH. When the pH had reached 6 - 7, the pH was raised a third time to about 10, which required 1.2 ml. 0.1 M NaOH. The sample was prepared by dissolving the pectin at 70°C and subsequently cooling the solution to the recording temperature. The temperature was maintained with a thermostatically controlled water bath.

1. Sample with DE=71%

Table 5.1: Multiple pH drop of pectin with DE=71%

| First dosage: 19 ml 0.1 M NaOH | | Second dosage: 1.1 ml 0.1 M NaOH | | Third dosage: 1.2 ml 0.1 M NaOH | |
|---|---|---|---|---|---|
| Time, minutes | pH | Time, minutes | pH | Time, minutes | pH |
| 0,2 | 10,4 | 0 | 10,08 | 0 | 10,22 |
| 0,5 | 10,21 | 0,5 | 9,88 | 2 | 9,63 |

(continued)

| First dosage: 19 ml 0.1 M NaOH | | Second dosage: 1.1 ml 0.1 M NaOH | | Third dosage: 1.2 ml 0.1 M NaOH | |
|---|---|---|---|---|---|
| Time, minutes | pH | Time, minutes | pH | Time, minutes | pH |
| 1 | 9,85 | 1 | 9,71 | 7 | 8,76 |
| 2 | 9,52 | 2 | 9,44 | 12 | 8,32 |
| 3 | 9,26 | 5 | 8,93 | 32 | 7,64 |
| 5 | 8,89 | 10 | 8,36 | 67 | 7,17 |
| 10 | 8,33 | 20 | 7,73 | 92 | 7,07 |
| 20 | 7,75 | 40 | 7,32 | 152 | 6,93 |
| 30 | 7,47 | 70 | 7,06 | 212 | 6,87 |
| 45 | 7,24 | 85 | 6,97 | 272 | 6,82 |
| 60 | 7,11 | 175 | 6,65 | 332 | 6,78 |
| 122 | 6,91 | 1140 | 6,38 | 402 | 6,74 |
| 181 | 6,85 | 1165 | 6,37 | 482 | 6,74 |
| 240 | 6,79 | | | | |
| 291 | 6,77 | | | | |
| 347 | 6,74 | | | | |
| 1298 | 6,32 | | | | |
| 1428 | 6,31 | | | | |
| 1508 | 6,3 | | | | |
| 1553 | 6,28 | | | | |

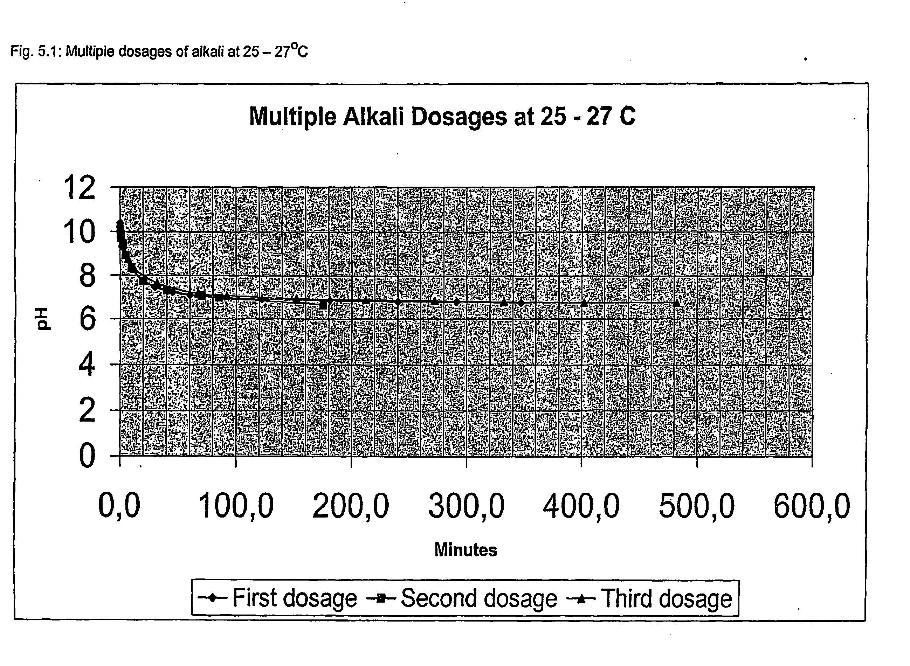

Fig. 5.1 shows that the rate of the pH-drop stays unchanged after at least three cycles, where the pH is first increased to about 10, then after the pH has dropped increased to about 10. After one cycle, the DE is decreased to about 66%, so the ability to continue reducing pH is caused by an incomplete de-esterification.

Thus, if alkalinity is appears in pulses, for at least three times pectin is able to reduce the alkali. In fact, in one experiment, which went on for seven days, a 200 ml. 1% pectin solution of DE=71% consumed 73 ml. of a 0.1 M NaOH solution. After this period, the DE has decreased to 9.1%.

Thus, 2 g. pectin consumes 7.3 mmol NaOH, which corresponds to about 0.3 g. NaOH. It also means that about 0.23 g. methanol is produced, which in combination with the acid effect of pectin may explain the anti-microbial effect of pectin.

Example 6: Effect of pectin concentration

The pH drop for one sample having DE=81.7% and made from dried lemon peel was recorded at a temperature of 30 - 32°C. The concentration of pectin was 0.05 - 2%. The sample was prepared by dissolving the pectin at 70°C and subsequently cooling the solution to the recording temperature. The temperature was maintained with a thermostatically controlled water bath.

1. Sample with DE=81.7%

Table 6.1: pH drop at different concentration of pectin solution with DE-81.7%

| 0.05% | | 0.1% | | 0.2% | | 0.5% | | 1.0% | | 2.0% | |
|---|---|---|---|---|---|---|---|---|---|---|---|
| Start pH=3.62 | | Start pH=3.70 | | Start pH=3.46 | | Start pH=3.15 | | Start pH=2.96 | | Start pH=2.87 | |
| 0.8 ml 0.1 M NaOH | | 0.8 ml 0.1 M NaOH | | 1.7 ml 0.1 M NaOH | | 4.4 ml 0.1 M NaOH | | 7.6 ml 0.1 M NaOH | | 15.5 ml 0.1 M NaOH | |
| Minutes | pH | Minutes | pH | Minutes | pH | Minutes | pH | Minutes | pH | Minutes | pH |
| 0 | 9,89 | 0 | 9,89 | 0 | 9,98 | 0 | 10,1 | 0 | 9,8 | 0 | 10,02 |
| 1 | 9,87 | 1 | 9,63 | 1 | 9,67 | 1 | 9,72 | 1 | 9,26 | 1 | 9,34 |
| 2 | 9,82 | 2 | 9,55 | 2 | 9,52 | 2 | 9,45 | 2 | 8,95 | 2 | 8,95 |
| 3 | 9,7 | 3 | 9,47 | 3 | 9,38 | 3 | 9,23 | 3 | 8,69 | 3 | 8,66 |
| 4 | 9,66 | 4 | 9,4 | 4 | 9,25 | 4 | 9,03 | 4 | 8,48 | 4 | 8,43 |
| 5 | 9,63 | 5 | 9,32 | 5 | 9,14 | 5 | 8,89 | 5 | 8,3 | 5 | 8,22 |
| 11 | 9,42 | 9 | 9,09 | 11 | 8,6 | 12 | 8,1 | 9 | 7,87 | 9 | 7,75 |
| 21 | 9,19 | 19 | 8,59 | 16 | 8,2 | 22 | 7,66 | 19 | 7,51 | 19 | 7,39 |
| 31 | 9 | 29 | 8,12 | 26 | 7,72 | 32 | 7,5 | 29 | 7,38 | 29 | 7,25 |
| 41 | 8,81 | 39 | 7,72 | 36 | 7,48 | 42 | 7,39 | 39 | 7,27 | 39 | 7,18 |
| 51 | 8,63 | 49 | 7,58 | 46 | 7,35 | 52 | 7,33 | 49 | 7,19 | 49 | 7,13 |
| 61 | 8,33 | 59 | 7,45 | 61 | 7,24 | 62 | 7,27 | 59 | 7,13 | 59 | 7,1 |

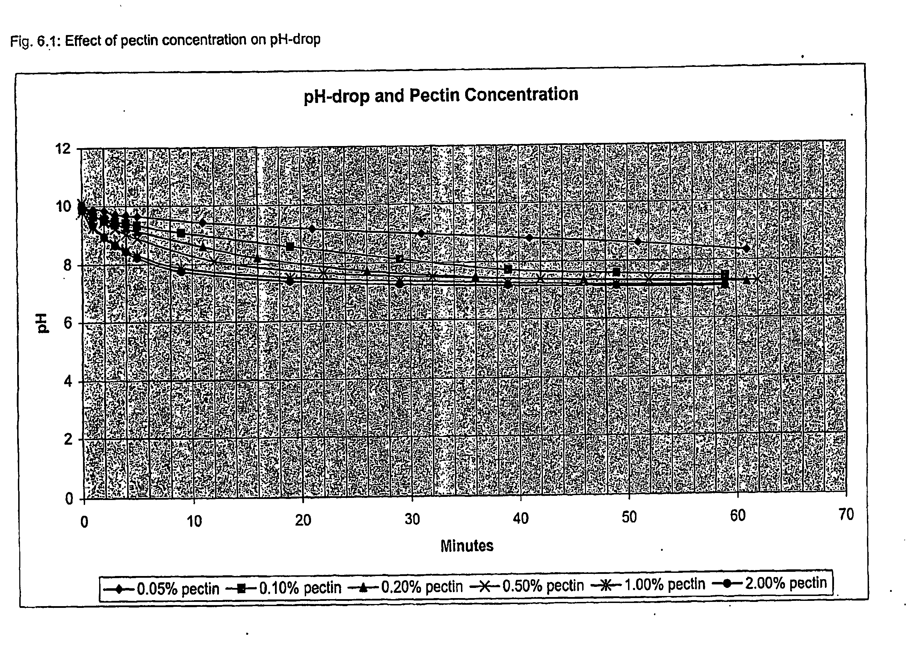

Fig. 6.1 shows that at pectin concentrations above 1%, the pH-drop appears to be independent of the pectin concentration. However, even at very low concentrations of pectin, a clear drop in pH occurs.

Example 7: pH drop of water

Carbon dioxide is soluble in water, and this experiment shows the pH drop of ion-exchanged water over time without the presence of pectin or other additions. The temperature of the water was kept at 25°C using a thermostatically controlled water bath.

1. Ion exchanged water

Table 7.1: pH drop of ion exchanged water

| Time, minutes | pH |
|---|---|
| 0 | 10,67 |
| 18 | 10,63 |
| 36 | 10,57 |
| 56 | 10,56 |
| 81 | 10,55 |
| 125 | 10,43 |
| 165 | 10,3 |
| 297 | 10,23 |
| 330 | 10,07 |

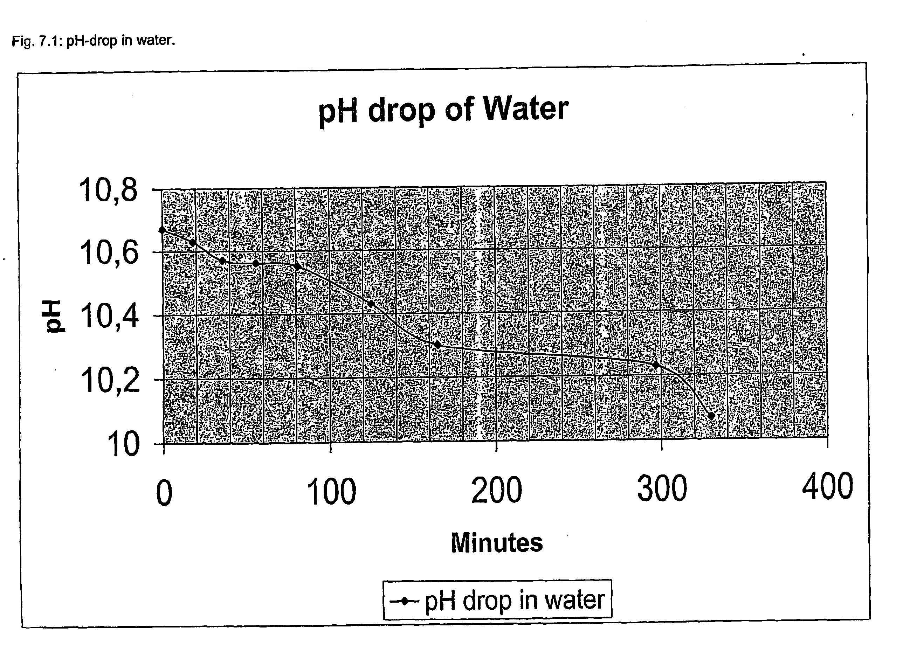

Fig. 7.1 shows that over a period of about 5 hours, the "natural" drop of pH in water is about 0.5 pH-units, so the error is tolerable.

Example 8: Propylene Glycol Alginate - Effect of esterification

Three samples with degree of esterification ranging from about 55 to about 85% were tested. All were titrated and the pH drop over time recorded for samples dissolved at 70°C and 20°C, respectively. The pH drop was measured at 30 - 32°C. Titration was done using 0.1008 M NaOH. The comment "unstable" refers to the pH-meter, which at high pH values showed an unstable reading.

1. Kelcoloid O. Esterification: High - about 85%

Table 8.1: Titration and pH drop of high DE PGA

| ml NaOH | pH | Comment | Time, minutes 70°C | pH | Time, minutes 20°C | pH |
|---|---|---|---|---|---|---|
| 0 | 3,89 | | 0 | 10 | 0 | 10,19 |
| 0,5 | 3,99 | | 1 | 7,77 | 1 | 7,74 |
| 1 | 4,1 | | 2 | 7,34 | 2 | 7,33 |
| 1,5 | 4,22 | | 3 | 7,14 | 3 | 7,13 |
| 2 | 4,38 | | 4 | 7 | 4 | 6,99 |
| 2,5 | 4,57 | | 5 | 6,89 | 5 | 6,86 |
| 3 | 4,89 | | 10 | 6,48 | 8 | 6,57 |
| 3,5 | 5,7 | | 15 | 6,2 | 38 | 5,41 |
| 4 | 8,82 | Unstable | 25 | 5,81 | 68 | 5,07 |
| | | | 53 | 5,29 | 132 | 4,77 |
| | | 70 | 70 | 5,12 | 1102 | 4,4 |
| | | | 90 | 4,99 | 1142 | 4,4 |
| | | | 116 | 4,89 | | |
| | | | 127 | 4,85 | | |

2. Manucol Ester ER/K. Esterification: High - about 80%.

Table 8.2: Titration and pH drop of high DE PGA

| ml NaOH | pH | Comment | Time, minutes 70°C | pH | Time, minutes 20°C | pH |
|---|---|---|---|---|---|---|
| 0 | 3,76 | | 0 | 10 | 0 | 10,2 |
| 0,5 | 3,82 | | 1 | 7,85 | 1 | 7,97 |
| 1 | 3,91 | | 2 | 7,38 | 2 | 7,44 |
| 1,5 | 4,01 | | 3 | 7,17 | 3 | 7,23 |
| 2 | 4,11 | | 4 | 7 | 4 | 7,08 |
| 2,5 | 4,24 | | 5 | 6,87 | 5 | 6,95 |
| 3 | 4,39 | | 7 | 6,66 | 9 | 6,58 |
| 3,5 | 4,58 | | 12 | 6,29 | 15 | 6,26 |
| 4 | 4,89 | | 17 | 6,03 | 31 | 5,75 |
| 4,5 | 5,63 | | 27 | 5,69 | 59 | 5,28 |

(continued)

| ml NaOH | pH | Comment | Time, minutes 70°C | pH | Time, minutes 20°C | pH |
|---|---|---|---|---|---|---|
| 5 | 8,88 | Unstable | 42 | 5,4 | 90 | 5,06 |
| | | | 57 | 5,24 | 142 | 4,87 |
| | | | | | 1114 | 4,54 |
| | | | | | 1163 | 4,53 |

Kelcoloid HVF. Esterification: Medium - about 55%

Table 8.3: Titration and pH drop of medium DE PGA

| ml NaOH | pH | Comment | Time, minutes 70°C | pH | Time, minutes 20°C | pH |
|---|---|---|---|---|---|---|
| 0 | 3,81 | | 0 | 10,21 | 0 | 10,29 |
| 0,5 | 3,85 | | 1 | 8,66 | 1 | 8,78 |
| 1 | 3,9 | | 2 | 7,98 | 2 | 8,07 |
| 1,5 | 3,95 | | 3 | 7,65 | 3 | 7,72 |
| 2 | 4 | | 4 | 7,47 | 4 | 7,51 |
| 2,5 | 4,06 | | 5 | 7,35 | 5 | 7,37 |
| 3 | 4,12 | | 7 | 7,16 | 7 | 7,16 |
| 3,5 | 4,19 | | 12 | 6,82 | 12 | 6,82 |
| 4 | 4,26 | | 27 | 6,3 | 27 | 6,27 |
| 4,5 | 4,34 | | 47 | 5,91 | 52 | 5,84 |
| 5 | 14,43 | | 67 | 5,69 | 77 | 5,63 |
| 5,5 | 4,53 | | 97 | 5,5 | 95 | 5,53 |
| 6 | 4,66 | | 152 | 5,31 | 1106 | 5,02 |
| 6,5 | 14,82 | | 222 | 5,19 | 1148 | 5,02 |
| 7 | 5,07 | | | | | |
| 7,5 | 5,56 | | | | | |
| 8 | 8,03 | Unstable | | | | |

4. Effect of multiple additions of alkali

The pH drop for one sample, Manucol Ester ER/K, was recorded at a temperature of 30 - 32°C. First, the pH was raised to about 10 with 4 ml. 0.1 M NaOH. When the sample had reached a pH of 5 - 6, the pH was again raised to about 10. This required 2.5 ml. 0.1 M NaOH. When the pH had reached 5 - 6, the pH was raised a third time to about 10, which required 2.0 ml. 0.1 M NaOH. When the pH had reached about 6, the pH was again increased to about 10, which required 1.5 ml. NaOH. The sample was prepared by dissolving the pectin at 70°C and subsequently cooling the solution to the recording temperature. The temperature was maintained with a thermostatically controlled water bath.

Table 8.4: Multiple pH drop of high DE PGA

| First dosage 4 ml 0.1 M NaOH | | Second dosage: 2.5 ml 0.1 M NaOH | | Third dosage: 2.0 ml 0.1 M NaOH | | Fourth dosage: 1.5 ml 0.1 M NaOH | |
|---|---|---|---|---|---|---|---|
| Time, minutes | pH | Time, minutes | pH | Time, minutes | pH | Time, minutes | pH |
| 0 | 10 | 0 | 10,24 | 0 | 9,89 | 0 | 9,97 |
| 1 | z7,85 | 1 | 8,29 | 1 | 8,26 | 1 | 8,7 |
| 2 | 7,38 | 2 | 7,62 | 2 | 7,64 | 2 | 8,04 |
| 3 | 7,17 | 3 | 7,36 | 3 | 7,37 | 3 | 7,67 |
| 4 | 7 | 4 | 7,2 | 4 | 7,21 | 4 | 7,47 |
| 5 | 6,87 | 5 | 7,07 | 5 | 7,09 | 5 | 7,33 |
| 7 | 6,66 | 9 | 6,64 | 9 | 6,7 | 11 | 6,84 |
| 12 | 6,29 | 14 | 6,29 | 13 | 6,45 | 16 | 6,56 |
| 17 | 6,03 | 19 | 6,04 | 18 | 6,23 | 22 | 6,31 |
| 27 | 5,69 | 24 | 5,85 | 23 | 6,06 | 31 | 6,03 |
| 42 | 5,4 | | | | | 147 | 5,13 |
| | | | 57 | 5,24 | | | |

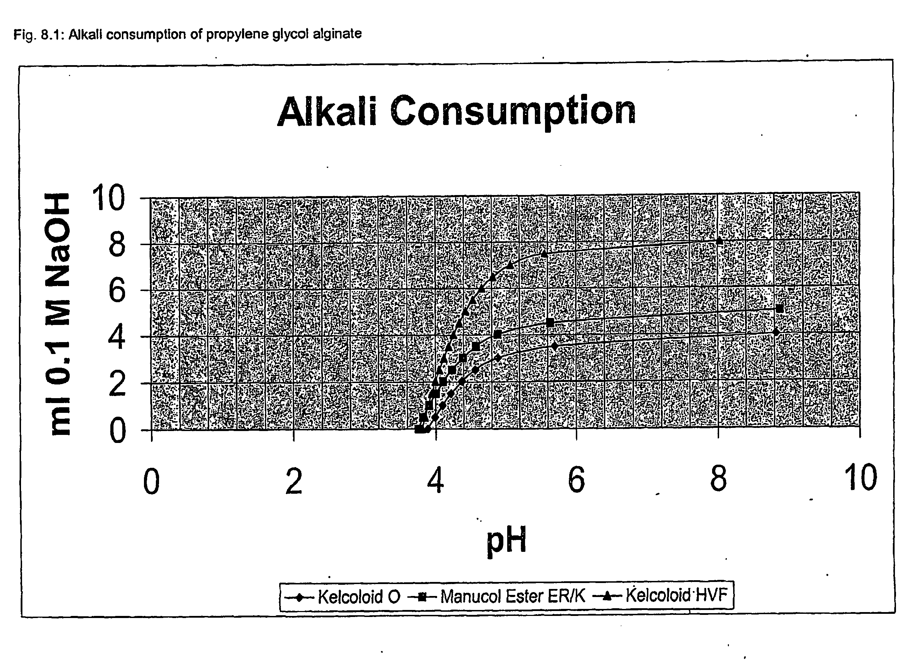

Fig. 8.1 shows that as the degree of esterifiaction increases in PGA, the less alkali can be consumed.

Buffer capacities are calculated to

- PGA with DE about 85%: About 4.1
- PGA with DE about 80%: About 5.7
- PGA with DE about 55%: About 8.1

Thus, PGA provides less buffering effect compared to pectin.

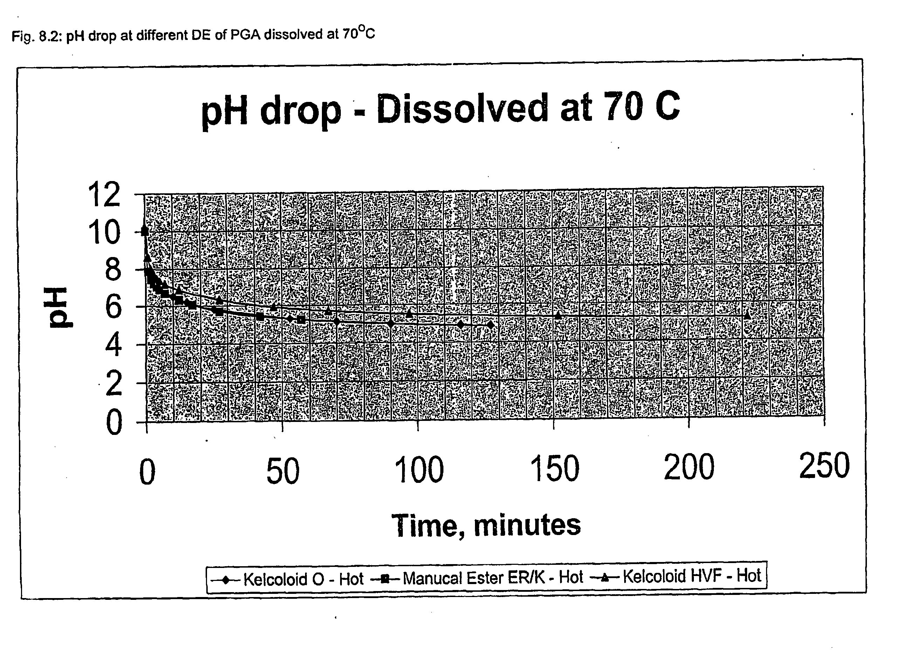

Fig. 8.2 shows that as for pectin, PGA provides a faster pH drop with increasing degree of esterification.

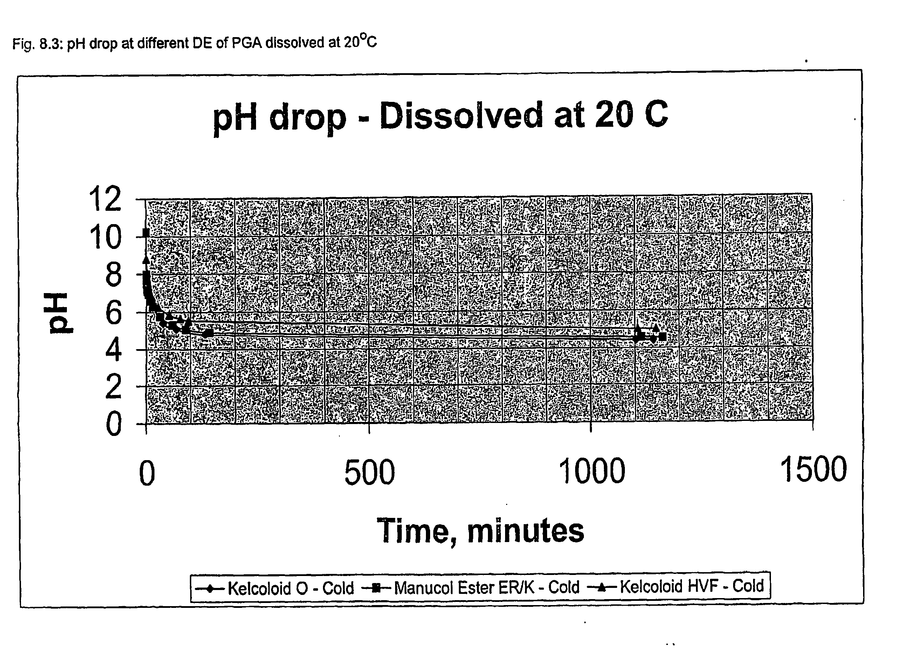

Fig. 8.3 shows the same dramatic influence of esterification even when the propylene glycol alginate is dissolved at 20°C. The figure shows that at the high DE, the pH is eventually decreased to below 5.

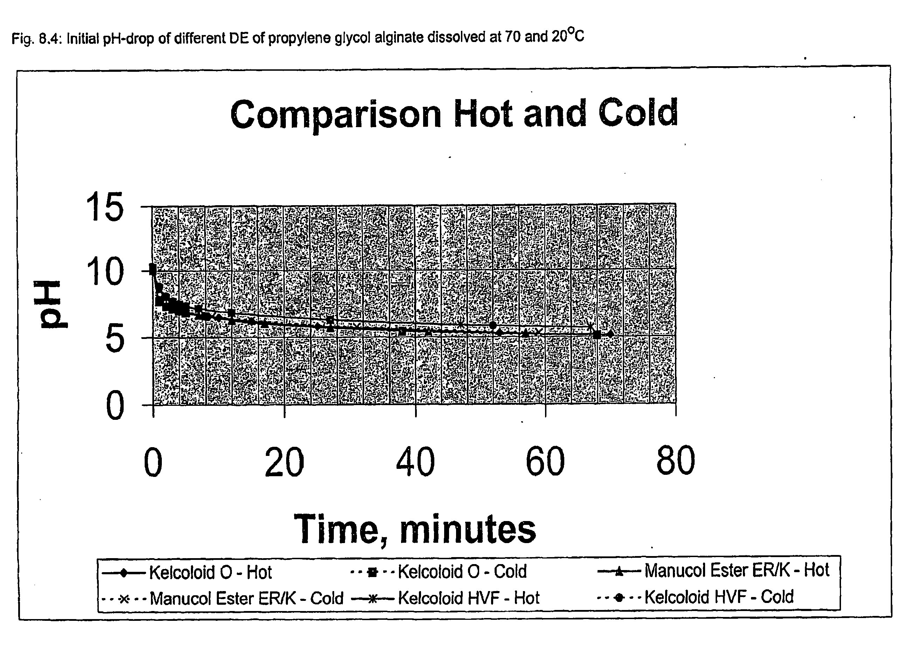

Fig. 8.4 shows that the pH-drop occurs to the same extent whether the propylene glycol alginate solution is made hot or cold.

Example 9: Effect of multiple additions of alkali to propylene glycol alginate

The pH drop for one sample, Manucol Ester ER/K, was recorded at a temperature of 30 - 32°C. First, the pH was raised to about 10 with 4 ml. 0.1 M NaOH. When the sample had reached a pH of 5 - 6, the pH was again raised to about 10. This required 2.5 ml. 0.1 M NaOH. When the pH had reached 5 - 6, the pH was raised a third time to about 10, which required 2.0 ml. 0.1 M NaOH. When the pH had reached about 6, the pH was again increased to about 10, which required 1.5 ml. NaOH. The sample was prepared by dissolving the pectin at 70°C and subsequently cooling the solution to the recording temperature. The temperature was maintained with a thermostatically controlled water bath.

Table 9.1: Multiple pH drop of high DE PGA

| First dosage 4 ml 0.1 M NaOH | | Second dosage: 2.5 ml 0.1 M NaOH | | Third dosage: 2.0 ml 0.1 M NaOH | | Fourth dosage: 1.5 ml 0.1 M NaOH | |
|---|---|---|---|---|---|---|---|
| Time, minutes | pH | Time, minutes | pH | Time, minutes | pH | Time, minutes | pH |
| 0 | 10 | 0 | 10,24 | 0 | 9,89 | 0 | 9,97 |
| 1 | 7,85 | 1 | 8,29 | 1 | 8,26 | 1 | 8,7 |
| 2 | 7,38 | 2 | 7,62 | 2 | 7,64 | 2 | 8,04 |

(continued)

| First dosage 4 ml 0.1 M NaOH | | Second dosage: 2.5 ml 0.1 M NaOH | | Third dosage: 2.0 ml 0.1 M NaOH | | Fourth dosage: 1.5 ml 0.1 M NaOH | |
|---|---|---|---|---|---|---|---|
| Time, minutes | pH | Time, minutes | pH | Time, minutes | pH | Time, minutes | pH |
| 3 | 7,17 | 3 | 7,36 | 3 | 7,37 | 3 | 7,67 |
| 4 | 7 | 4 | 7,2 | 4 | 7,21 | 4 | 7,47 |
| 5 | 6,87 | 5 | 7,07 | 5 | 7,09 | 5 | 7,33 |
| 7 | 6,66 | 9 | 6,64 | 9 | 6,7 | 11 | 6,84 |
| 12 | 6,29 | 14 | 6,29 | 13 | 6,45 | 16 | 6,56 |
| 17 | 6,03 | 19 | 6,04 | 18 | 6,23 | 22 | 6,31 |
| 27 | 5,69 | 24 | 5,85 | 23 | 6,06 | 31 | 6,03 |
| 42 | 5,4 | | | | | 147 | 5,13 |
| | | 57 | | 5,24 | | | |

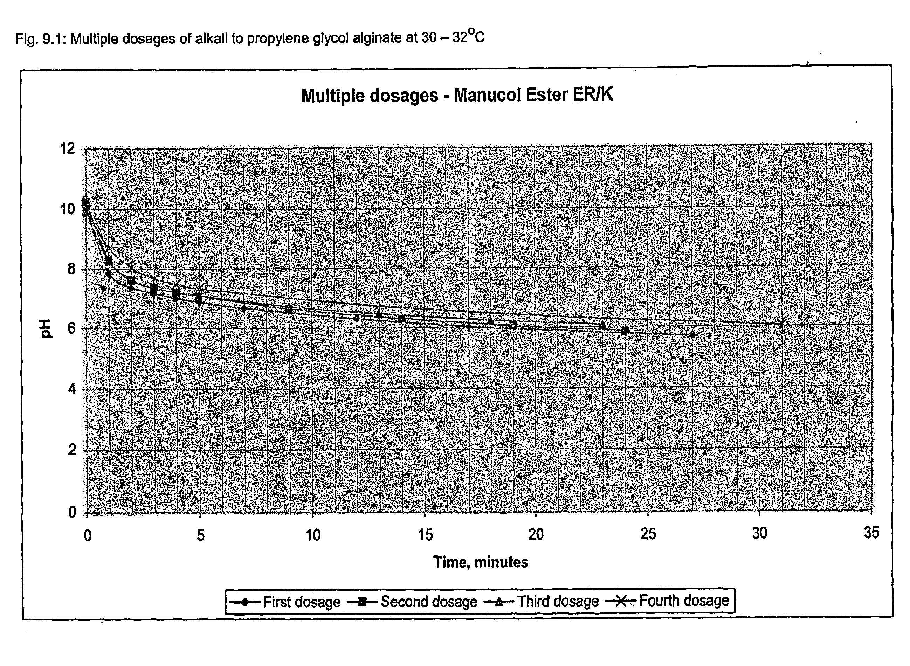

Fig. 9.1 shows a tendency for the pH-drop to become slower after two cycles.

Example 10: pH-drop in lotion

The pH drop in lotions made according to the two methods described in "Materials and Methods" section 2.1 were measured using pectin of about DE=81.7%.

10 grams lotion was slurried in 50 ml distilled water and pH was adjusted with 0.1 M NaOH to about 10. Pectin concentration in slurry: 0.125%. Temperature: 30°C.

Table 10.1: pH-drop of lotions

| Method 1 | | Method 2 | |
|---|---|---|---|
| Minutes | pH | Minutes | pH |
| 0 | 9,98 | 0 | 10,24 |
| 1 | 9,84 | 1 | 10,07 |
| 2 | 9,78 | 2 | 9,97 |
| 3 | 9,68 | 3 | 9,89 |
| 4 | 9,63 | 4 | 9,83 |
| 5 | 9,58 | 5 | 9,78 |
| 17 | 9,28 | 10 | 9,59 |
| 32 | 9,15 | 25 | 9,38 |
| 50 | 9,04 | 40 | 9,24 |
| 62 | 9 | 55 | 9,15 |

It may seem that when pectin is dissolved in the water phase before mixing with the oil phase provides for a more rapid pH-drop. However, when taking into consideration, that the curve for pectin dissolved in the water phase starts at a slightly lower pH, the two curves are close to identical. Thus, there is nothing to suggest that one of the methods for making the lotion influences the effect of the pectin.

The lotions were tested by 12 persons - 6 females and 6 males, with the following remarks from the test persons:

- Easy to spread on the skin
- Non-sticky

- Non-greasy
- Softens skin within one minute after application
- Skin-softening remains for at least 24 hours
- Removes skin-itching within one minute after application
- Skin-itching does not reoccur within 24 hours
- Athlete's foot is effectively combated for at least 24 hours

The lotion was also tested on one dog, which had developed a rash on the nose. Treatment of the nose with the lotion twice for one day reduced the rash visibly. To similar treatments over the next two days reduced to rash to an extent, where the rash was difficult to see.

Example 11: pH-drop of cloth

Cloths were prepared according to the method in "Materials and Methods" section 2.m.

Table 11.1: pH-drop of cloth soaked in a 0.01% pectin solution

| 0.01% pectin | | | | | | | |
|---|---|---|---|---|---|---|---|
| Mw=123,000 | | Mw=95,000 | | Mw=41,500 | | Mw=25,000 | |
| Minutes | pH | Minutes | pH | Minutes | pH | Minutes | pH |
| 0 | 11 | 0 | 11 | 0 | 11 | 0 | 11 |
| 20 | 9 | 20 | 9 | 20 | 9 | 20 | 9 |
| 140 | 8,5 | 140 | 8,5 | 140 | 8,5 | 140 | 8,5 |
| 290 | 8 | 290 | 8 | 290 | 8 | 290 | 8 |
| 500 | 7,5 | 500 | 7,5 | 500 | 7,5 | 500 | 7,5 |

Table 11.2: pH-drop of cloth soaked in a 0.05% pectin solution

| 0.05% pectin | | | | | | | |
|---|---|---|---|---|---|---|---|
| Mw=123,000 | | Mw=95,000 | | Mw=41,500 | | Mw=25,000 | |
| Minutes | pH | Minutes | pH | Minutes | pH | Minutes | pH |
| 0 | 11 | 0 | 11 | 0 | 11 | 0 | 11 |
| 20 | 9 | 20 | 9 | 20 | 9 | 20 | 9 |
| 140 | 8,5 | 140 | 8,5 | 140 | 8,5 | 140 | 8,5 |
| 290 | 8 | 290 | 8 | 290 | 8 | 290 | 8 |
| 500 | 7,5 | 500 | 7,5 | 500 | 7,5 | 500 | 7,5 |

Table 11.3: pH-drop of cloth soaked in a 0.10% pectin solution

| 0.10% pectin | | | | | | | |
|---|---|---|---|---|---|---|---|
| Mw=123,000 | | Mw=95,000 | | Mw=41,500 | | Mw=25,000 | |
| Minutes | pH | Minutes | pH | Minutes | pH | Minutes | pH |
| 0 | 11 | 0 | 11 | 0 | 11 | 0 | 11 |
| 20 | 9 | 20 | 9 | 20 | 9 | 20 | 9 |
| 140 | 8,5 | 140 | 8,5 | 140 | 8,5 | 140 | 8,5 |
| 290 | 8 | 290 | 8 | 290 | 8 | 290 | 8 |
| 500 | 7,5 | 500 | 7,5 | 500 | 7,5 | 500 | 7,5 |

Table 11.4: pH-drop of cloth soaked in a 0.20% pectin solution

| 0.20% pectin | | | | | | | |
|---|---|---|---|---|---|---|---|
| Mw=123,000 | | Mw=95,000 | | Mw=41,500 | | Mw=25,000 | |
| Minutes | pH | Minutes | pH | Minutes | pH | Minutes | pH |
| 0 | 11 | 0 | 10 | 0 | 11 | 0 | 11 |
| 20 | 9 | 20 | 8,5 | 20 | 8,5 | 20 | 9 |
| 140 | 8,5 | 140 | 8 | 140 | 8 | 140 | 8,5 |
| 290 | 8 | 290 | 7,5 | 290 | 8 | 290 | 8 |
| 500 | 7,5 | 500 | 7 | 500 | 7,5 | 500 | 7,5 |

Table 11.5: pH-drop of cloth soaked in a 0.50% pectin solution

| 0.50% pectin | | | | | | | |
|---|---|---|---|---|---|---|---|
| Mw=123,000 | | Mw=95,000 | | Mw=41,500 | | Mw=25,000 | |
| Minutes | pH | Minutes | pH | Minutes | pH | Minutes | pH |
| 3 | 10 | 0 | 10 | 0 | 10 | 0 | 10 |
| 20 | 8,5 | 20 | 8,5 | 20 | 8,5 | 20 | 8,5 |
| 140 | 8 | 140 | 8 | 140 | 8 | 140 | 8 |
| 290 | 7,5 | 290 | 7,5 | 290 | 7,5 | 290 | 7,5 |
| 500 | 7 | 500 | 7 | 500 | 7 | 500 | 7 |

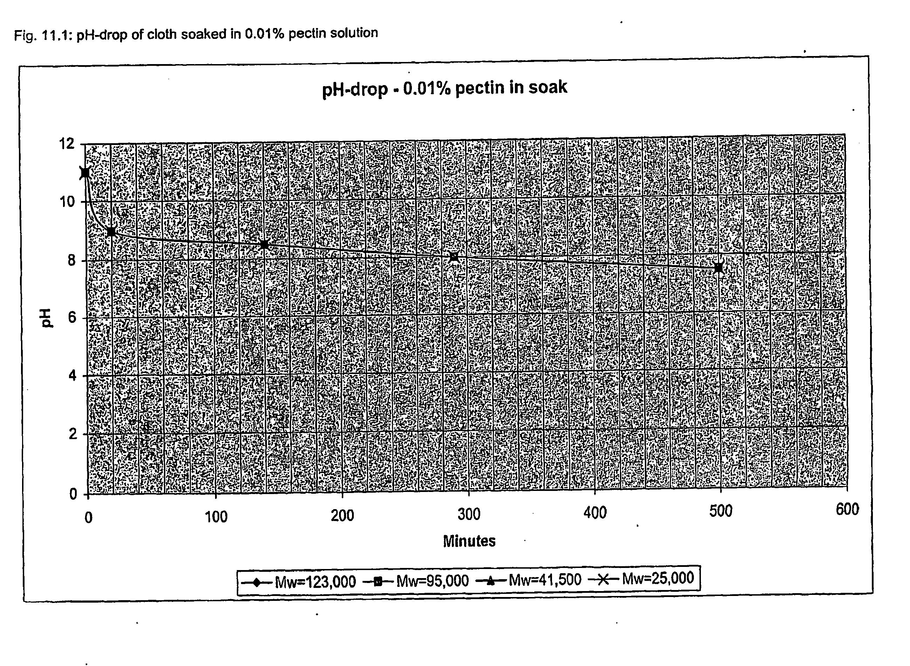

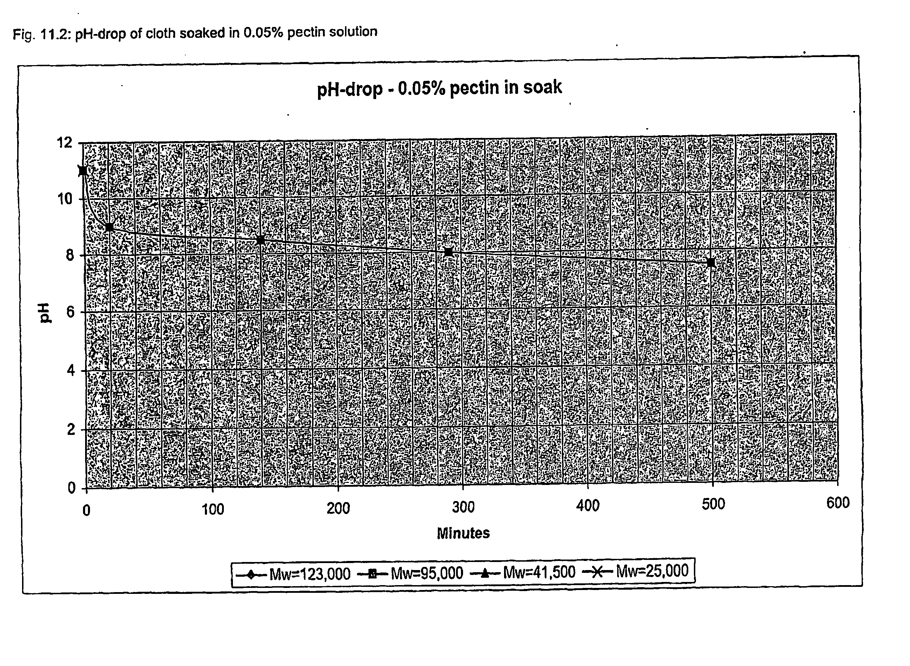

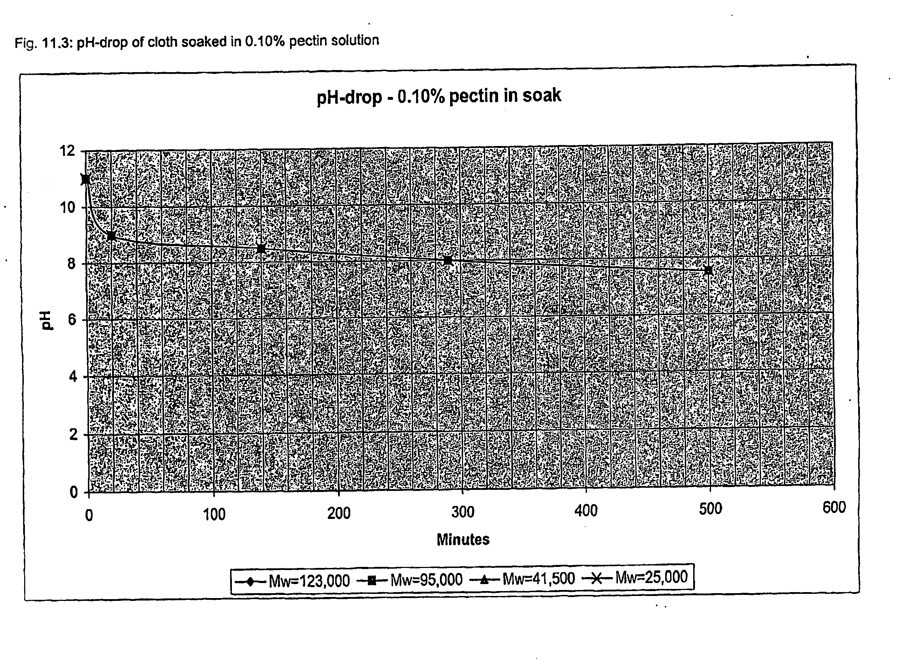

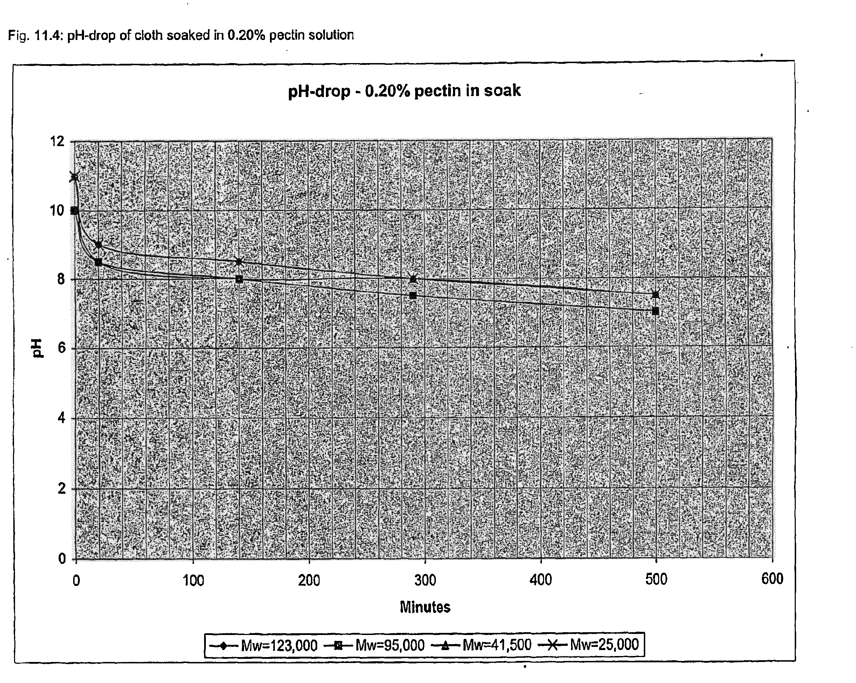

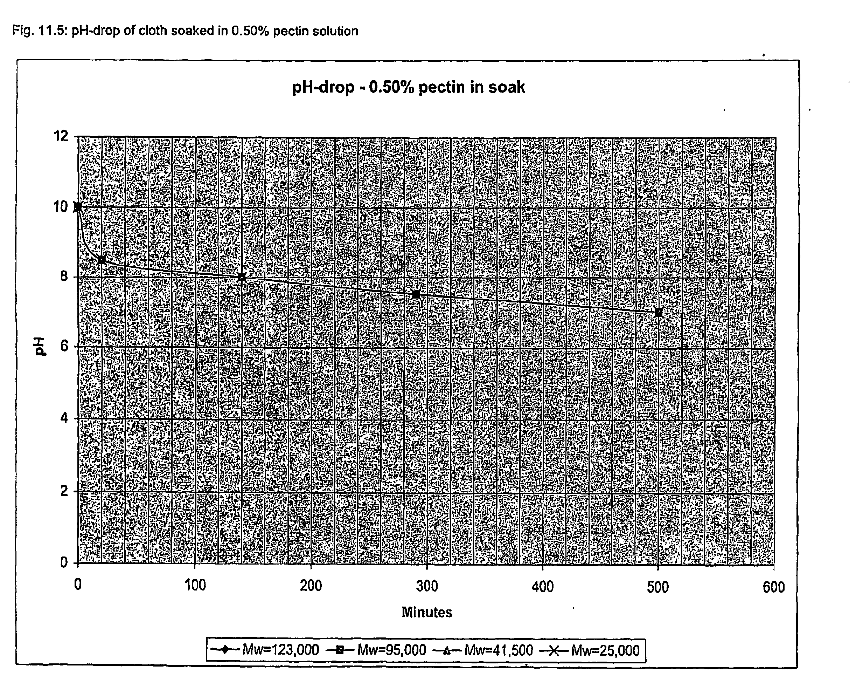

Fig. 11.1 - 11.5 show that irrespective of the concentration of pectin during soaking, and irrespective of the molecular weight of the pectin, the pH-drop is quite similar.

However, when the cloth is soaked in a pectin solution, the dried cloth becomes stiffer. Table 11.1 shows this effect:

Table 11.1: Stiffness of cloth as a function of pectin concentration in soak and molecular weight of pectin

| | Mw | | | |
|---|---|---|---|---|
| Pectin % in soak | 123,000 | 95,000 | 41,500 | 25,000 |
| 0,01 | Soft | Soft | Soft | Soft |
| 0,05 | Slightly soft | Soft | Soft | Soft |
| 0,1 | Acceptable | Soft | Soft | Soft |
| 0,2 | Stiff | Accepable | Soft | Soft |
| 0,5 | Too stiff | Stiff | Acceptable | Acceptable |

Table 11.1 shows that as the molecular weight decreases, the cloth can contain more pectin without becoming unacceptably stiff.

Mw=123,000 becomes unacceptably stiff at concentrations in the soak above 0.10% Mw=95,000 becomes unacceptably stiff at concentrations in the soak above 0.20% Mw=41,500 and Mw=25,000 become unacceptably stiff at concentrations in the soak above 0.50%.

A rinse is normally performed using 16 liters of water. Assuming that the rinse dosage is 100 ml, then 0.01% pectin in the rinse corresponds to a pectin solution of 1.57%. 0.05% pectin in the rinse corresponds to a pectin solution of 7.4%. 0.10% pectin in the rinse corresponds to a pectin solution of 13.79%. 0.20% pectin in the rinse corresponds to a pectin solution of 26.47% and 0.05% pectin in the rinse corresponds to a pectin solution of 44.44%.

The effect on Brookfield viscosity of such pectin solutions are shown in table 11.2:

Table 11.2: Viscosity of different molecular weights of pectin at various concentrations

| Sample | % | Viscosity, cP | Comment |
|---|---|---|---|
| Mw=123,00 | 1,6 | 229 | |
| | 4 | 12880 | Not fully dissolved |
| Mw=95,000 | 1,6 | 99,5 | |
| | 4 | 2840 | Not fully dissolved |
| Mw=41,500 | 1,6 | 11,3 | |
| | 4 | 73,6 | |
| | 8 | 790 | |
| | 12 | 19200 | Not fully dissolved |
| Mw=25,000 | 1,6 | 7,8 | |
| | 4 | 29,2 | |
| | 8 | 270 | |
| | 12 | 3560 | Thick but dissolved |
| | 16 | 26800 | Not fully dissolved |

It is clear that as the molecular weights drops, it becomes easier to dissolve the pectin, and in addition the viscosity becomes lower. This enables a rinse to contain more pectin in lower rinse dosage.

For pectin with a molecular weight of 123,000, the maximum concentration of pectin in the rinse is about 2%, for a pectin with a molecular weight of 95,000, the maximum concentration of pectin in the rinse is about 3%, for a pectin with molecular weight of 41,500, the maximum concentration of pectin in the rinse is about 10% and for a pectin with molecular weight of 25,000, the maximum concentration of pectin in the rinse is about 12%.

Example 12: Effect of blending pectin products

Pectin products having a DE of 93.4% and 9.6%, respectively were blended 1:1 and 100 g. 1% solution was prepared of the blend through heating to 70 °C. The consumption of alkali at 25 °C and the pH-drop over time at 30 - 32 °C was recorded. Titration was done using 0.1008 M NaOH. The comment "unstable" refers to the pH-meter, which at high pH values showed an unstable reading.

Table 12: Titration and pH drop of pectin blends

| ml. NaOH | pH | Comment | Time, minutes | pH |
|---|---|---|---|---|
| 0 | 4,26 | | 0 | 10,00 |
| 1 | 4,27 | | 1 | 9,31 |
| 2 | 4,33 | | 2 | 8,98 |
| 3 | 4,40 | | 3 | 8,76 |
| 4 | 4,48 | | 4 | 8,58 |
| 5 | 4,56 | | 5 | 8,44 |
| 6 | 4,64 | | 16 | 7,66 |
| 7 | 4,74 | | 40 | 7,33 |
| 8 | 4,85 | | 49 | 7,22 |
| 9 | 14,97 | | 69 | 7,04 |
| 10 | 5,12 | | | |
| 11 | 5,33 | | | |

(continued)

| ml. NaOH | pH | Comment | Time, minutes | pH |
|---|---|---|---|---|
| 12 | 5,66 | | | |
| 13 | 6,82 | Slightly unstable | | |
| 14 | 9,73 | Unstable | | |

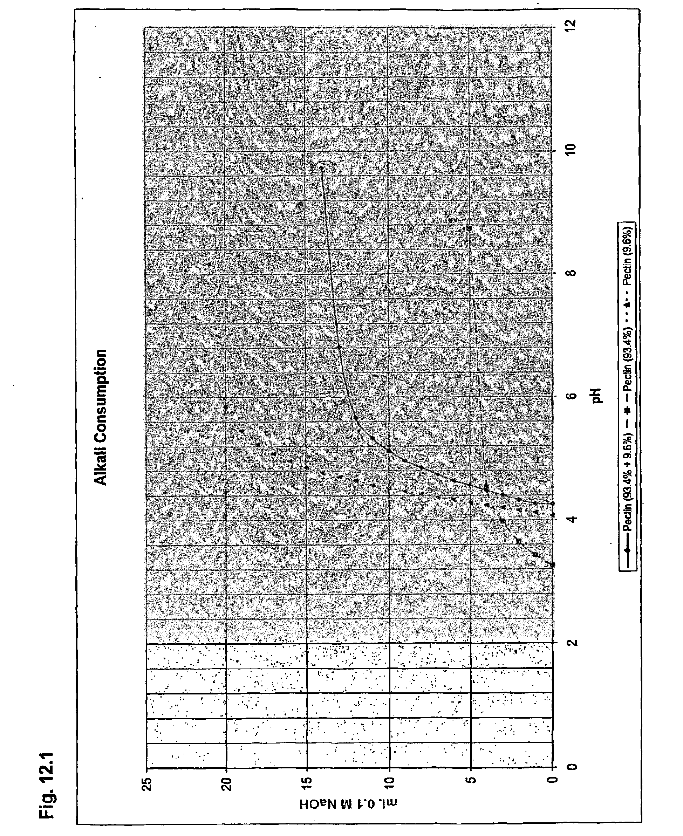

Fig. 12.1 shows that blending high DE pectin and low DE pectin results in an alkali consumption in between the alkali consumption of the individual pectin products.

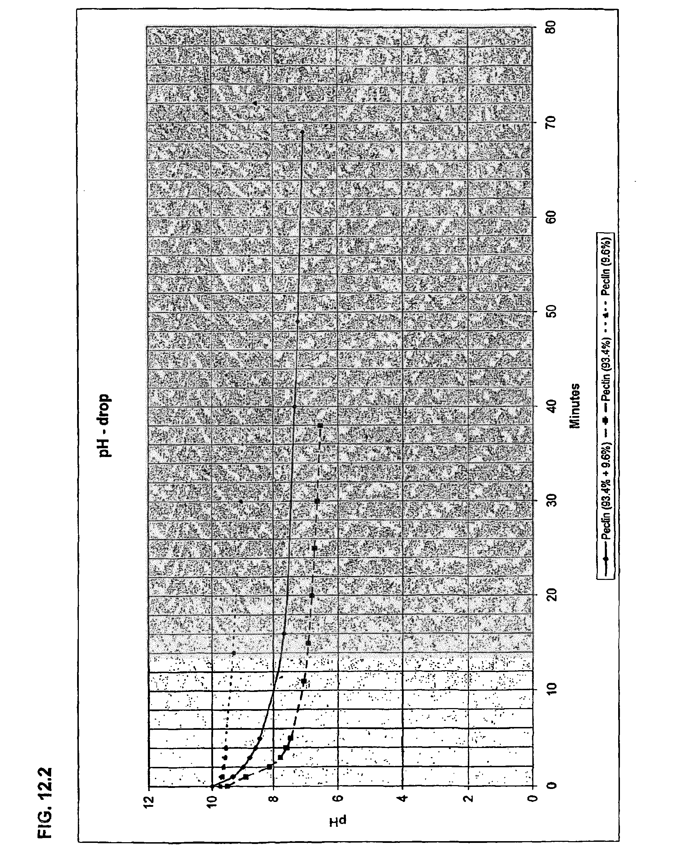

Fig. 12.2 shows that the pH drop over time falls between the pH drop over time of the individual components.

Compared to the individual components, the blend of high DE pectin and low DE pectin provides for an increase in alkali consumption compared to pure high DE pectin and an increase in pH-drop compared to low DE pectin.

Example 13: Effect of blending high ester pectin and low ester propylene glycol alginate.

A blend of 50% of a pectin having a DE of 93.4% and 50% of a propylene glycol alginate (PGA) having a DE of 55% was dissolved at 70 % in a similar manner as in example 12 and compared with the alkali consumption of the individual components.

Table 13: Titration and pH drop of blend of high ester pectin and low ester propylene glycol alginate

| ml. NaOH | pH | Remarks | Time, minutes | pH |
|---|---|---|---|---|
| 0 | 3,66 | | 0 | 10,00 |
| 1 | 3,77 | | 1 | 9,24 |
| 2 | 3,9 | | 2 | 8,51 |
| 3 | 4,05 | 3 | 3 | 8,05 |
| 4 | 4,22 | 4 | 4 | 7,76 |
| 5 | 4,46 | | 5 | 7,57 |
| 6 | 4,83 | | 7 | 7,34 |
| 7 | 6,47 | Slightly unstable | 18 | 6,79 |
| 8 | 9,89 | Unstable | 28 | 6,55 |
| | | | 97 | 5,87 |

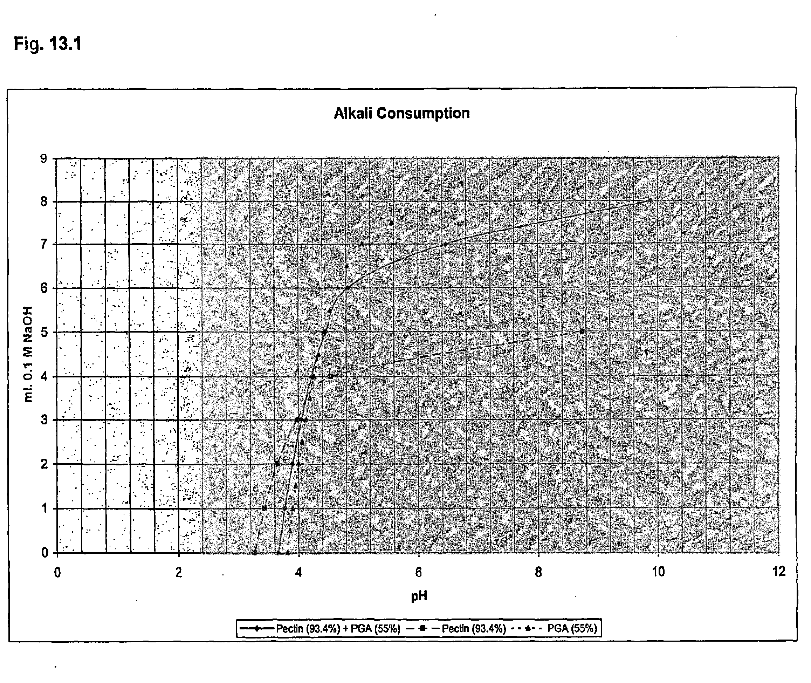

Fig. 13.1 shows that the alkali consumption falls between the alkali consumption of the individual components, but the use of a mixture of a high DE pectin and a medium DE PGA results in a smaller increase in alkali consumption than observed with the mixture of a high DE pectin and a low DE pectin of example 12.

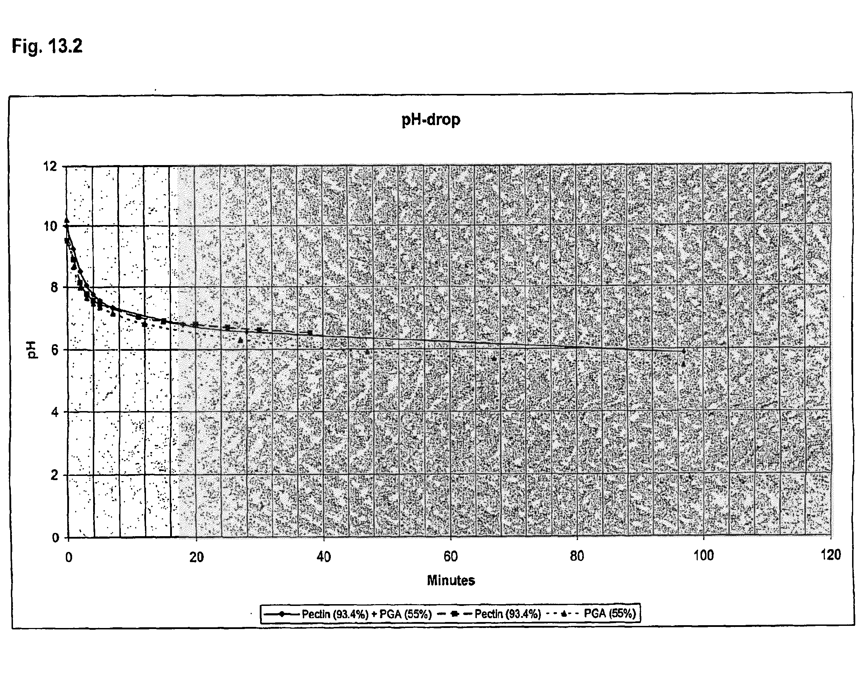

Fig. 13.2 shows that the pH-drop of the blend falls between the pH-drop of the individual components. However, even a relatively low esterified PGA provides for a faster pH-drop than a much higher esterified pectin.

Compared with the individual components the blend provides an increase in alkali consumption compared to the pectin product alone.

Example 14: Effect of blending high De propylene glycol alginate and low DE pectin.

A blend of 50% of a propylene glycol alginate (PGA) having a DE of 85% and 50% of a pectin having a DE of 9.6% was dissolved at 70% in a similar manner as in example 12 and compared with the alkali consumption of the individual components.

Table 14: Titration and pH drop of blend of high ester propylene glycol alginate and low ester pectin

| ml. NaOH | pH | Remarks | Time, minutes | pH |
|---|---|---|---|---|
| 0 | 4,06 | | 0 | 10 |

(continued)

| ml. NaOH | pH | Remarks | Time, minutes | pH |
|---|---|---|---|---|
| 1 | 4,12 | | 1 | 9,04 |
| 2 | 4,18 | 2 | 2 | 8,55 |
| 3 | 4,25 | 3 | 3 | 8,22 |
| 4 | 4,33 | 4 | 4 | 7,97 |
| 5 | 4,4 | | 5 | 7,79 |
| 6 | 4,49 | 20 | 20 | 6,9 |
| 7 | 4,57 | 34 | 34 | 6,6 |
| 8 | 4,68 | 44 | 44 | 6,47 |
| 9 | 4,8 | 69 | 69 | 6,25 |
| 10 | 4,94 | | 93 | 6,12 |
| 11 | 5,13 | | | |
| 12 | 5,41 | | | |
| 13 | 6,5 | Unstable | | |
| 14 | 9,29 | Unstable | | |

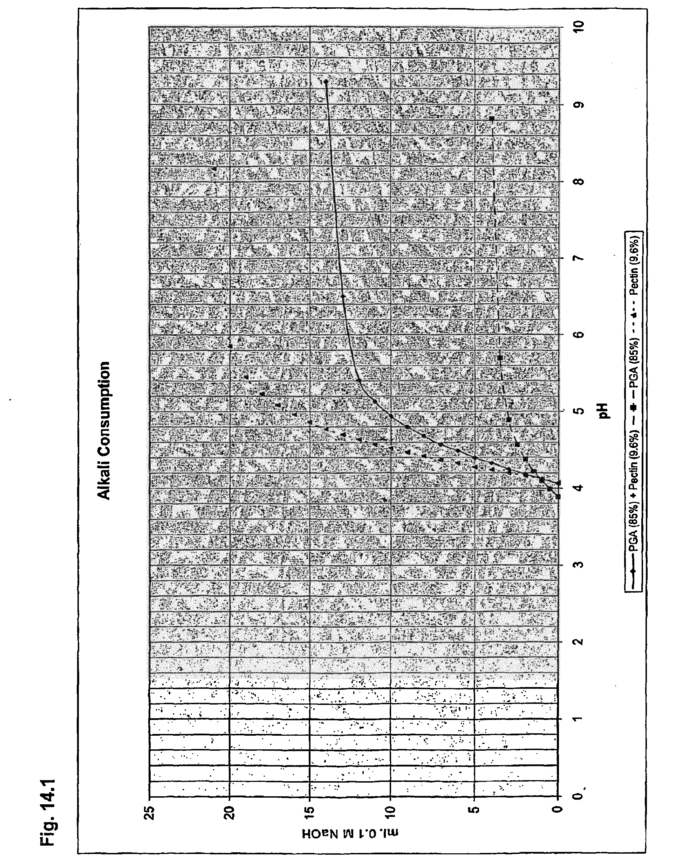

Fig. 14.1 shows that the alkali consumption of the blend falls in between the alkali consumption of the individual components.

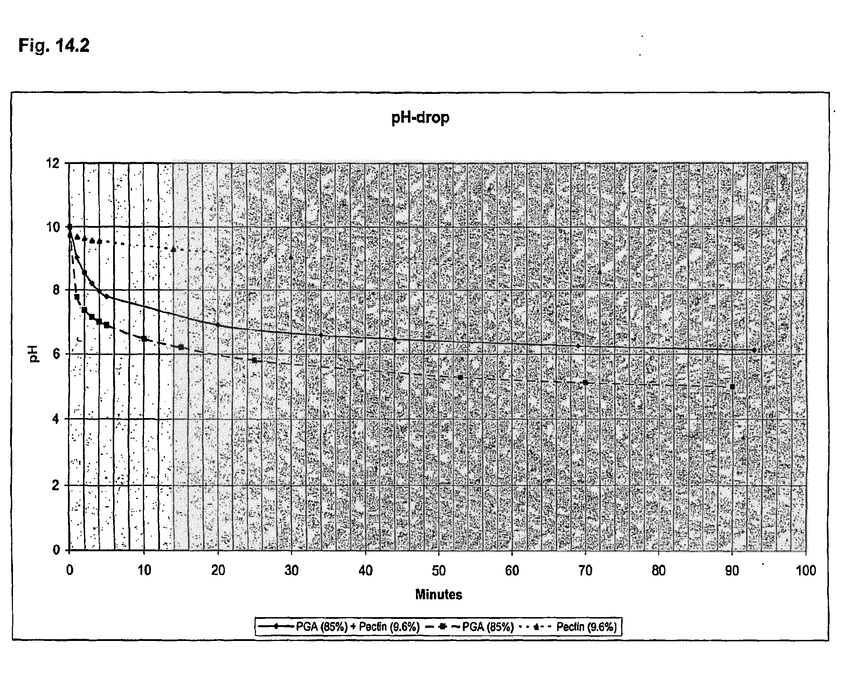

Fig. 14.2 shows that the pH drop over time falls between the pH-drop of the individual components.

Compared to the individual components, the blend provides for an increase in alkali consumption compared to propylene glycol alginate alone, and an increase in pH drop compared to low DE pectin alone.

## Claims

1. A skin-protecting alkalinity-controlling composition comprising a mixture of at least one high DE pectin ester having a degree of esterification (DE) in the range from about 70% to about 100%, and at least one low DE carboxylic acid polysaccharide having a degree of esterification (DE) in the range from about 5% to about 40%.

2. The composition according to claim 1 wherein said low DE carboxylic acid polysaccharide has a degree of esterification (DE) from about 10% to about 35%.

3. The composition according to claims 1 or 2 wherein the pectin ester is selected from the group consisting of aliphatic, arylaliphatic,
cycloaliphatic or heterocyclic alcohols.

4. The composition according to claim 3 wherein the pectin ester is selected from the group consisting of an ester of methanol, ethanol, propanol or isopropanol.

5. The composition according to claim 4 wherein the pectin ester is an ester of methanol.

6. The composition according to any one of the preceding claims wherein the pectin has a molecular weight in the range from about 5,000 to about 140,000, preferably in the range from about 10,000 to about 125,000, most preferably in the range from about 10,000 to about 40,000.

7. The composition according to claim 1, wherein the ester groups of the high DE pectin esters and the said low DE polysaccharides are distributed in a block-wise fashion.

8. The composition according to claim 1, wherein the ester groups of the high DE pectin esters and the said low DE polysaccharides are distributed in a random fashion.

**9.** The composition according to any one of the preceding claims in a form suitable for use on human skin.

**10.** The composition according to any one of the preceding claims 1-8 in a form suitable for use on animal skin.

**11.** The composition according to any one of the preceding claims 1-8 in the form of a product selected from the group consisting of skin creams, skin lotions and soap products.

**12.** A composition as claimed in any one of claims 1-11 for use in protecting the skin acid mantle by controlling the alkalinity.

**13.** Use of a composition comprising a mixture of at least one high DE pectin ester having a degree of esterification (DE) in the range from about 70% to about 100%, and at least one low DE carboxylic acid polysaccharide having a degree of esterification (DE) in the range from about 5% to about 40% for the preparation of a medicament for protecting the skin acid mantle by controlling alkalinity.

**14.** The use according to claim 13 wherein the medicament is in a form suitable for use on human skin.

**15.** The use according to claim 13 wherein the medicament is in a form suitable for use on animal skin.

**16.** The use according to claim 13 wherein the medicament is in the form of a product selected from the group consisting of skin creams, skin lotions and soap products.

**17.** Use according to claim 13 wherein the medicament is incorporated into articles which are in contact with the skin.

**18.** Use according to claim 17 wherein the articles are selected from the group consisting of female hygiene products, diapers, ostomy products, wound care products, lotionized tissue products, fabric treating products, and laundry rinse products.

**Patentansprüche**

**1.** Eine hautschützende, Alkalinität kontrollierende Zusammensetzung, die eine Mischung aus mindestens einem Pektinester mit hohem Veresterungsgrad mit einem Grad an Veresterung (DE) im Bereich von etwa 70 % bis etwa 100 % und mindestens einem Carbonsäurepolysaccharid mit niedrigem Veresterungsgrad mit einem Grad an Veresterung (DE) im Bereich von etwa 5 % bis etwa 40 % beinhaltet.

**2.** Zusammensetzung gemäß Anspruch 1, wobei das Carbonsäurepolysaccharid mit niedrigem Veresterungsgrad einen Grad an Veresterung (DE) von etwa 10 % bis etwa 35 % aufweist.

**3.** Zusammensetzung gemäß Anspruch 1 oder 2, wobei der Pektinester aus der Gruppe ausgewählt ist, die aus aliphatischen, arylaliphatischen, cycloaliphatischen oder heterocyclischen Alkoholen besteht.

**4.** Zusammensetzung gemäß Anspruch 3, wobei der Pektinester aus der Gruppe ausgewählt ist, die aus einem Ester von Methanol, Ethanol, Propanol oder Isopropanol besteht.

**5.** Zusammensetzung gemäß Anspruch 4, wobei der Pektinester ein Ester von Methanol ist.

**6.** Zusammensetzung gemäß einem der vorhergehenden Ansprüche, wobei das Pektin ein Molekulargewicht im Bereich von etwa 5000 bis etwa 140000, vorzugsweise im Bereich von etwa 10000 bis etwa 125000, am besten im Bereich von etwa 10000 bis etwa 40000 aufweist.

**7.** Zusammensetzung gemäß Anspruch 1, wobei die Estergruppen der Pektinester mit hohem Veresterungsgrad und die Polysaccharide mit niedrigem Veresterungsgrad auf eine blockartige Weise verteilt sind.

**8.** Zusammensetzung gemäß Anspruch 1, wobei die Estergruppen der Pektinester mit hohem Veresterungsgrad und die Polysaccharide mit niedrigem Veresterungsgrad auf eine willkürliche Weise verteilt sind.

**9.** Zusammensetzung gemäß einem der vorhergehenden Ansprüche in einer Form, die zur Verwendung auf menschlicher Haut geeignet ist.

**10.** Zusammensetzung gemäß einem der vorhergehenden Ansprüche 1-8 in einer Form, die zur Verwendung auf Tierhaut geeignet ist.

**11.** Zusammensetzung gemäß einem der vorhergehenden Ansprüche 1-8 in der Form eines Produkts, ausgewählt aus der Gruppe, die aus Hautcremes, Hautlotionen und Seifenprodukten besteht.

**12.** Zusammensetzung gemäß einem der Ansprüche 1-11 zur Verwendung beim Schutz des Hautsäuremantels durch Kontrollieren der Alkalinität.

**13.** Verwendung einer Zusammensetzung, die eine Mischung aus mindestens einem Pektinester mit hohem Veresterungsgrad mit einem Grad an Veresterung (DE) im Bereich von etwa 70 % bis etwa 100 % und mindestens einem Carbonsäurepolysaccharid mit niedrigem Veresterungsgrad mit einem Grad an Veresterung (DE) im Bereich von etwa 5 % bis etwa 40 % zur Zubereitung eines Medikaments zum Schutz des Hautsäuremantels durch Kontrollieren der Alkalinität beinhaltet.

**14.** Verwendung gemäß Anspruch 13, wobei das Medikament in einer Form ist, die zur Verwendung auf menschlicher Haut geeignet ist.

**15.** Verwendung gemäß Anspruch 13, wobei das Medikament in einer Form ist, die zur Verwendung auf Tierhaut geeignet ist.

**16.** Verwendung gemäß Anspruch 13, wobei das Medikament in der Form eines Produkts ist, ausgewählt aus der Gruppe, die aus Hautcremes, Hautlotionen und Seifenprodukten besteht.

**17.** Verwendung gemäß Anspruch 13, wobei das Medikament in Artikeln eingeschlossen ist, die in Kontakt mit der Haut sind.

**18.** Verwendung gemäß Anspruch 17, wobei die Artikel aus der Gruppe ausgewählt sind, die aus Folgendem besteht: Produkten für die weibliche Hygiene, Windeln, Stomaprodukten, Produkten zur Wundversorgung, Gewebeprodukten mit Lotion, Produkten zur Behandlung von Textilerzeugnissen und Produkten zum Auswaschen von Wäsche.

## Revendications

**1.** Une composition corrigeant l'alcalinité et protégeant la peau comprenant un mélange d'au moins un ester de pectine à DE élevé ayant un degré d'estérification (DE) dans la gamme allant d'environ 70 % à environ 100 %, et d'au moins un polysaccharide d'acide carboxylique à faible DE ayant un degré d'estérification (DE) dans la gamme allant d'environ 5 % à environ 40 %.

**2.** La composition selon la revendication 1 dans laquelle ledit polysaccharide d'acide carboxylique à faible DE a un degré d'estérification (DE) allant d'environ 10 % à environ 35 %.

**3.** La composition selon les revendications 1 ou 2 dans laquelle l'ester de pectine est sélectionné dans le groupe consistant en alcools aliphatiques, arylaliphatiques, cycloaliphatiques ou hétérocycliques.

**4.** La composition selon la revendication 3 dans laquelle l'ester de pectine est sélectionné dans le groupe consistant en un ester de méthanol, d'éthanol, de propanol ou d'isopropanol.

**5.** La composition selon la revendication 4 dans laquelle l'ester de pectine est un ester de méthanol.

**6.** La composition selon une quelconque des revendications précédentes dans laquelle la pectine a une masse moléculaire dans la gamme allant d'environ 5 000 à environ 140 000, préférablement dans la gamme allant d'environ 10 000 à environ 125 000, des plus préférablement dans la gamme allant d'environ 10 000 à environ 40 000.

**7.** La composition selon la revendication 1, dans laquelle les groupes ester des esters de pectine à DE élevé et desdits polysaccharides à faible DE sont répartis à la manière de blocs.

8. La composition selon la revendication 1, dans laquelle les groupes ester des esters de pectine à DE élevé et desdits polysaccharides à faible DE sont répartis de manière aléatoire.

9. La composition selon l'une quelconque des revendications précédentes sous une forme appropriée pour une utilisation sur la peau humaine.

10. La composition selon l'une quelconque des revendications précédentes 1 à 8 sous une forme appropriée pour une utilisation sur la peau d'un animal.

11. La composition selon l'une quelconque des revendications précédentes 1 à 8 sous la forme d'un produit sélectionné dans le groupe consistant en des crèmes pour la peau, des lotions pour la peau et produits de savon.

12. Une composition telle que revendiquée dans l'une quelconque des revendications 1 à 11 destinée pour une utilisation pour protéger le manteau acide de la peau en corrigeant l'alcalinité.

13. Utilisation d'une composition comprenant un mélange d'au moins un ester de pectine à DE élevé ayant un degré d'estérification (DE) dans la gamme allant d'environ 70 % à environ 100 %, et d'au moins un polysaccharide d'acide carboxylique à faible DE ayant un degré d'estérification (DE) dans la gamme allant d'environ 5 % à environ 40 % pour la préparation d'un médicament destiné à protéger le manteau acide de la peau en corrigeant l'alcalinité.

14. L'utilisation selon la revendication 13 dans laquelle le médicament est sous une forme appropriée pour une utilisation sur la peau humaine.

15. L'utilisation selon la revendication 13 dans laquelle le médicament est sous une forme appropriée pour une utilisation sur la peau d'un animal.

16. L'utilisation selon la revendication 13 dans laquelle le médicament est sous la forme d'un produit sélectionné dans le groupe consistant en des crèmes pour la peau, des lotions pour la peau et des produits de savon.

17. L'utilisation selon la revendication 13 dans laquelle le médicament est incorporé dans des articles qui sont en contact avec la peau.

18. L'utilisation selon la revendication 17 dans laquelle les articles sont sélectionnés dans le groupe consistant en des produits hygiéniques féminins, des couches, des produits pour stomie, des produits de soin des plaies, des produits pour mouchoirs enrichis de lotion, des produits pour le traitement de tissus, et des produits de rinçage pour le linge. MURG * 110969

Fig. 1.1: Alkali consumption at different molecular weight

Alkali consumption and Molecular Weight

ml.0.1 M NaOH

pH

123.000
108.500
95.000
71.500
41.500

Molecular Weight and pH-Drop - Dissolution at 70 C
pH
12
10
8
6
4
2
0
0
500
1000
1500
2000
Minutes
123.000
108.500
95.000
71.500
41.500

Fig. 1.2: pH-drop over time for pectin dissolved at 70°C.

Molecular Weight and pH-drop - Dissolution at 20 C
pH
12
10
8
6
4
2
0
0
50
100
150
200
250
300
Minutes
123.000
108.500
95.000
71.500
41.000

Fig. 1.3: pH-drop over time for pectin dissolved at 20°C.

Fig 2.1: Alkali consumption at different DE.

Alkali Consumption and DE

ml. 0.1 M NaOH

0
10
20
30
40
50

0
2
4
6
8
10
12

pH

DE=9.6% DE=34.4% DE=71% DE=93.4%

Fig 2.2: pH-drop at different DE of pectin dissolved at 70°C

DE and pH-drop - Dissolved at 70 C
pH
12
10
8
6
4
2
0
0
1000
2000
3000
4000
5000
Minutes
DE=9.6%
DE=34.4%
DE=71%
DE=93.4%

DE and pH-drop - Dissolved at 20 C
pH
12
10
8
6
4
2
0
-500
0
500
1000
1500
Minutes
DE=9.6%
DE=34.4%
DE=71%
DE=93.4%

Fig. 2.3: pH-drop different DE of pectin dissolved at 20$^{o}$C.

Fig. 2.4: Initial pH-drop of different DE of pectin dissolved at 70°C and 20°C.

pH-drop and DE - Hot and Cold

DE=9.6% Hot
DE=34.4% Hot
DE=71% Hot
DE=93.4% Hot
DE=9.6% Cold
DE=34.4% Cold
DE=71% Cold
DE=93.4% Cold

Fig. 3.1: Alkali consumption random and blocky DE pectin.

Alkali Consumption and Ester Distribution
ml. 0.1 M NaOH
35
30
25
20
15
10
5
0
0
2
4
6
8
10
pH
Random
Block

pH-drop - Hot and Cold - Random and Blocky
pH
15
10
5
0
0
500
1000
1500
Minutes
Random Hot
Block Hot
Random Cold
Block Cold

Fig. 3.2: pH-drop of pectin with different ester group distribution dissolved at 70 and 20°C.

pH-drop and Ester Distribution - First about 100 minutes
pH
12
10
8
6
4
2
0
0
50
100
150
Minutes
Random
Block

Fig. 3.3: Initial pH-drop of pectin solutions with different ester group distribution.

Fig. 4.1: pH-drop at various temperatures.

pH drop with time
pH
12
10
8
6
4
2
0
0,0
100,0
200,0
300,0
400,0
500,0
Time, minutes
T=25-27C
T=30-32C
T=45-47C

Multiple Alkali Dosages at 25 - 27 C
pH
12
10
8
6
4
2
0
0,0
100,0
200,0
300,0
400,0
500,0
600,0
Minutes
First dosage
Second dosage
Third dosage

Fig. 5.1: Multiple dosages of alkali at 25 – 27°C

pH-drop and Pectin Concentration
pH
12
10
8
6
4
2
0
Minutes
0
10
20
30
40
50
60
70
0.05% pectin
0.10% pectin
0.20% pectin
0.50% pectin
1.00% pectin
2.00% pectin

Fig. 6.1: Effect of pectin concentration on pH-drop

pH drop of Water
pH
10,8
10,6
10,4
10,2
10
0
100
200
300
400
Minutes
pH drop in water

Fig. 7.1: pH-drop in water.

Fig. 8.1: Alkali consumption of propylene glycol alginate

Alkali Consumption
ml 0.1 M NaOH
10
8
6
4
2
0
0
2
4
6
8
10
pH
Kelcoloid O
Manucol Ester ER/K
Kelcoloid HVF

Fig. 8.2: pH drop at different DE of PGA dissolved at 70°C

pH drop - Dissolved at 70 C

pH

Time, minutes

Kelcoloid O - Hot
Manucal Ester ER/K - Hot
Kelcoloid HVF - Hot

Fig. 8.3: pH drop at different DE of PGA dissolved at 20°C

pH drop - Dissolved at 20 C
pH
12
10
8
6
4
2
0
0
500
1000
1500
Time, minutes
Kelcoloid O - Cold
Manucol Ester ER/K - Cold
Kelcoloid HVF - Cold

Fig. 8.4: Initial pH-drop of different DE of propylene glycol alginate dissolved at 70 and 20°C

Comparison Hot and Cold
pH
15
10
5
0
0
20
40
60
80
Time, minutes
Kelcoloid O - Hot
Kelcoloid O - Cold
Manucol Ester ER/K - Hot
Manucol Ester ER/K - Cold
Kelcoloid HVF - Hot
Kelcoloid HVF - Cold

Fig. 9.1: Multiple dosages of alkali to propylene glycol alginate at 30 – 32°C

Multiple dosages - Manucol Ester ER/K
12
10
8
6
4
2
0
pH
0
5
10
15
20
25
30
35
Time, minutes
First dosage
Second dosage
Third dosage
Fourth dosage

pH-drop of Lotion
pH
10,5
10
9,5
9
8,5
0
20
40
60
80
Minutes
Pectin in water phase
Pectin in oil phase

Fig. 10.1: pH-drop of lotions

pH-drop - 0.01% pectin in soak
pH
12
10
8
6
4
2
0
0
100
200
300
400
500
600
Minutes
Mw=123,000
Mw=95,000
Mw=41,500
Mw=25,000

Fig. 11.1: pH-drop of cloth soaked in 0.01% pectin solution

Fig. 11.2: pH-drop of cloth soaked in 0.05% pectin solution

pH-drop - 0.05% pectin in soak
pH
12
10
8
6
4
2
0
0
100
200
300
400
500
600
Minutes
Mw=123,000
Mw=95,000
Mw=41,500
Mw=25,000

pH-drop - 0.10% pectin in soak
pH
12
10
8
6
4
2
0
0
100
200
300
400
500
600
Minutes
Mw=123,000
Mw=95,000
Mw=41,500
Mw=25,000

Fig. 11.3: pH-drop of cloth soaked in 0.10% pectin solution

pH-drop - 0.20% pectin in soak
pH
12
10
8
6
4
2
0
0
100
200
300
400
500
600
Minutes
Mw=123,000
Mw=95,000
Mw=41,500
Mw=25,000

Fig. 11.4: pH-drop of cloth soaked in 0.20% pectin solution

pH-drop - 0.50% pectin in soak
pH
12
10
8
6
4
2
0
0
100
200
300
400
500
600
Minutes
Mw=123,000
Mw=95,000
Mw=41,500
Mw=25,000

Fig. 11.5: pH-drop of cloth soaked in 0.50% pectin solution

Fig. 12.1

Alkali Consumption
ml. 0.1 M NaOH
25
20
15
10
5
0
0
2
4
6
8
10
12
pH
Pectin (93.4% + 9.6%)
Pectin (93.4%)
Pectin (9.6%)

FIG. 12.2

pH - drop
pH
12
10
8
6
4
2
0
0
10
20
30
40
50
60
70
80
Minutes
Pectin (93.4% + 9.6%)
Pectin (93.4%)
Pectin (9.6%)

Fig. 13.1

Alkali Consumption
ml. 0.1 M NaOH
0
1
2
3
4
5
6
7
8
9
pH
0
2
4
6
8
10
12
Pectin (93.4%) + PGA (55%)
Pectin (93.4%)
PGA (55%)

Fig. 13.2

pH-drop
pH
12
10
8
6
4
2
0
0
20
40
60
80
100
120
Minutes
Pectin (93.4%) + PGA (55%)
Pectin (93.4%)
PGA (55%)

Fig. 14.1

Alkali Consumption
mL 0.1 M NaOH
pH
PGA (85%) + Pectin (9.6%)
PGA (85%)
Pectin (9.6%)

Fig. 14.2

pH-drop
pH
12
10
8
6
4
2
0
Minutes
0
10
20
30
40
50
60
70
80
90
100
PGA (85%) + Pectin (9.6%)
PGA (85%)
Pectin (9.6%)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5929051 A, Ni
- US 1634879 A, Nanji
- US 2375376 A, Maclay
- WO 2004020472 A
- WO 8912648 A
- US 2132065 A
- US 3982003 A
- US 4831127 A
- WO 9115517 A
- US 5354851 A
- US 5403612 A
- US 5567462 A
- US 5656734 A
- WO 9749734 A
- US 5384400 A
- US 6624298 B
- US 6605715 B
- US 5859217 A
- US 5416205 A
- US 5837254 A
- US 5972321 A
- US 4666707 A
- US 6589923 B
- US 4335025 A
- US 6551987 B
- US 6013618 A
- US 5626852 A
- US 6352700 B
- US 6150405 A
- US 5667769 A
- US 4761279 A
- US 2253389 A
- GB 541528 A
- US 2478170 A
- JP 2001226220 B
- WO 0214374 A
- WO 04005352 A
- US 6509311 B
- US 6428837 B
- US 4278657 A
- WO 02055034 A
- EP 664300 A
- WO 2004026317 A
- US 5942478 A
- WO 0228447 A
- US 4813942 A


### Non-patent literature cited in the description


- **DUMITRIU, S.** Polysaccharides, Structural diversity and functional versatility. Marcel Dekker, Inc, 1998, 416-419
- **KERTESZ, Z. I.** The Pectic Substances. Interscience Publishers, Inc, 1951
- **TESSLER, M. M. ; BILIMERS, R. L.** Preparation of Starch Esters. *Journal of Environmental Polymer Degradation,* 1996, vol. 4, 85-89
- The Pectic Substances. Interscience Publishers, Inc, 1951
- Quantitative organic analysis via functional groups. Propylene glycol alginate - Quantitative determination of the ester groups is carried out by the saponification method. John Wiley and Sons Publication, 169-172